# EUROPÄISCHE PATENTSCHRIFT

(11) **EP 1 768 962 B1**
(45) Veröffentlichungstag und Bekanntmachung des Hinweises auf die Patenterteilung: **11.08.2010**
(21) Anmeldenummer: 05750710.5
(22) Anmeldetag: 17.06.2005
(51) Int. Cl.: C07D 233/58, C07D 213/06, C07F 9/02, C07F 9/54, C07C 209/84

(54) **VERFAHREN ZUR HERSTELLUNG VON ONIUM-SALZEN MIT GERINGEM CHLORID-GEHALT**
METHOD FOR PRODUCING ONIUM SALTS WITH A LOW CHLORIDE CONTENT
PROCEDE POUR PRODUIRE DES SELS D'ONIUM A FAIBLE TENEUR EN CHLORURE

(30) Priorität: 16.07.2004 DE 102004034543
(43) Veröffentlichungstag der Anmeldung: 04.04.2007
(73) Patentinhaber: Merck Patent GmbH, 64293 Darmstadt (DE)
(72) Erfinder: IGNATYEV, Nikolai (Mykola), 47058 Duisburg (DE); WELZ-BIERMANN, Urs, 64646 Heppenheim (DE); BARTHEN, Peter, 47495 Rheinberg (DE); WILLNER, Helge, 45481 Muelheim/Ruhr (DE)
(86) Internationale Anmeldenummer: PCT/EP2005/006550
(87) Internationale Veröffentlichungsnummer: WO 2006/007912

(56) Entgegenhaltungen:
- WO-A-00/16902
- WO-A-03/002579
- NISHIDA T ET AL: "Physical and electrochemical properties of 1-alkyl-3-methylimidazolium tetrafluoroborate for electrolyte" JOURNAL OF FLUORINE CHEMISTRY, ELSEVIER SEQUOIA, LAUSANNE, CH, Bd. 120, Nr. 2, 1. April 2003 (2003-04-01), Seiten 135-141, XP004413662 ISSN: 0022-1139 in der Anmeldung erwähnt
- HOLBREY J D ET AL: "The phase behaviour of 1-alkyl-3-methylimidazolium tetrafluoroborates; ionic liquids and ionic liquid crystals" JOURNAL OF THE CHEMICAL SOCIETY, SECTION A: INORGANIC, PHYSICAL AND THEORETICAL CHEMISTRY, CHEMICAL SOCIETY. LETCHWORTH, GB, Nr. 13, Juli 1999 (1999-07), Seiten 2133-2139, XP002181156
- DAVIS, JAMES H., JR. ET AL: "Synthesis and purification of ionic liquids" 2003, IONIC LIQUIDS IN SYNTHESIS , 7-21. EDITOR(S): WASSERSCHEID, PETER; WELTON, THOMAS. PUBLISHER: WILEY-VCH VERLAG GMBH & CO. KGAA, WEINHEIM, GERMANY. CODEN: 69DUHH; ISBN: 3-527-30515-7 , XP002342206 Seite 16 - Seite 19

## Beschreibung

Die Erfindung betrifft ein Verfahren zur Herstellung von Onium-Salzen nach Anspruch 1.

Eine große Anzahl von Onium-Salzen sind ionische Flüssigkeiten. Ionische Flüssigkeiten stellen durch Ihre Eigenschaften in der modernen Forschung eine wirksame Alternative zu traditionellen flüchtigen organischen Solventien für die organische Synthese dar. Die Verwendung von ionischen Flüssigkeiten als neues Reaktionsmedium könnte weiterhin eine praktische Lösung sowohl für die Lösungsmittel Emission als auch für Probleme in der Wiederaufbereitung von Katalysatoren sein.

Übersichtsartikel zu ionischen Flüssigkeiten sind beispielsweise R. Sheldon "Catalytic reactions in ionic liquids", Chem. Commun., 2001, 2399-2407; M.J. Earle, K.R. Seddon "Ionic liquids. Green solvent for the future", Pure Appl. Chem., 72 (2000), 1391-1398; P. Wasserscheid, W. Keim "Ionische Flüssigkeiten - neue Lösungen für die Übergangsmetallkatalyse", Angew. Chem., 112 (2000), 3926-3945; T. Welton "Room temperature, ionic liquids. Solvents for synthesis and catalysis", Chem. Rev., 92 (1999), 2071-2083 oder R. Hagiwara, Ya. Ito "Room temperature ionic liquids of alkylimidazolium cations and fluoroanions", J. Fluorine Chem., 105 (2000), 221-227).

Ionische Flüssigkeiten oder flüssige Salze sind ionische Spezies, die aus einem organischen Kation und einem in der Regel anorganischen Anion bestehen. Sie enthalten keine neutralen Moleküle und weisen meistens Schmelzpunkte kleiner 373 K auf. Der Schmelzpunkt kann jedoch auch höher liegen, ohne die Anwendbarkeit der Salze auf allen Anwendungsgebieten zu beschränken. Beispiele für organische Kationen sind unter anderem Tetraalkylammonium, Tetraalkylphosphonium, N-Alkylpyridinium, 1,3-Dialkylimidazolium oder Trialkylsulfonium. Unter einer Vielzahl von geeigneten Anionen sind beispielsweise BF₄-, PF₆-, SbF₆-, NO₃⁻, CF₃SO₃⁻, (CF₃SO₂)₂N⁻, ArylSO₃⁻, CF₃CO₂⁻, CH₃CO₂⁻ oder Al₂Cl₇⁻ zu nennen.

Die Eigenschaften der ionischen Flüssigkeiten, beispielsweise der Schmelzpunkt, die thermische und elektrochemische Stabilität oder die Viskosität, werden durch die Auswahl der Kationen und Anionen bestimmt. Ionische Flüssigkeiten sind nicht flüchtige Materialien und können daher nicht durch herkömmliche Methoden der Aufreinigung, wie beispielsweise der Destillation, gereinigt werden, wie sie für die meisten organischen Lösungsmittel entwickelt wurden.

Daher ist bei Verfahren zur Herstellung von Onium-Salzen, insbesondere ionischer Flüssigkeiten, die Technologie von entscheidender Bedeutung, damit diese durch die Reaktion per se oder die Reaktionsführung mit geringen Verunreinigungen synthetisiert werden können. Eine vorwiegend vorhandene Verunreinigung in bekannten ionischen Flüssigkeiten sind Halogenid-Ionen. Ist der Anteil an Halogenid-Ionen, beispielsweise ChloridIonen, größer als 1000 ppm (0,1 %), so reduziert sich die Anwendbarkeit der ionischen Flüssigkeit, insbesondere in der Anwendung für elektrochemische Prozesse.

Aufgabe der vorliegenden Erfindung war dementsprechend ein alternatives Verfahren zur Herstellung von Onium-Salzen mit geringem Chlorid-Gehalt zur Verfügung zu stellen, welches zu Produkten mit hoher Reinheit in guter Ausbeute führt und auch für eine großtechnische Produktion geeignet ist.

Die Aufgabe wird durch das erfindungsgemäße Verfahren gelöst.

Das erfindungsgemäße Verfahren ist eine Verbesserung der bekannten Syntheseverfahren, die in der Regel 2-Stufen-Verfahren sind, wie in P. Wasserscheid und W. Keim, Angew. Chem. 112 (2000), 3926-3945 beschrieben. Im ersten Schritt der bekannten Verfahren wird eine organische Base, typischerweise ein Amin, Phosphin oder eine heterocyclische Verbindung, mit einem Alkylhalogenid alkyliert und das entstehende Halogenid im zweiten Schritt über einen Anionenaustausch in das gewünschte Salz umgewandelt.

Typischerweise werden auf diesem Wege ionische Flüssigkeiten mit dem Tetrafluoroborat-Anion hergestellt, wobei das Halogenid, beispielsweise 1-Ethyl-3-methyl-imidazolium-chlorid oder -bromid, in der zweiten Stufe mit NaBF₄ in Aceton nach S. Park und R. J. Kazlauskas, J. Organic Chemistry, 66 (2001), 8395-8401, mit NaBF₄ in Wasser nach R. Karmakar und A. Samanta, J. Phys. Chem. A, 106 (2002), 6670-6675, mit AgBF₄ oder HBF₄ in Wasser nach J. D. Holbrey und K. R. Seddon, J. Chem. Soc., Dalton Trans., (1999), 2133-2139, mit NH₄BF₄ in Aceton nach J. Fuller et al, J. Electrochem. Soc., 144 (1997), 3881-3885, mit HBF₄ in Methanol nach T. Nishida et al, J. of Fluorine Chem., 120 (2003), 135-141 oder mit NH₄BF₄ unter Mikrowellenbestrahlung nach V.V. Namboodiri und R. S. Varma, Tetrahedron Lett., 43 (2002), 5381-5383 umgesetzt werden kann.

WO 00/16902 beschreibt ein Verfahren zur Herstellung von 1-Butyl-3-methylimidazoliumhydrogensulfat durch Umsetzung eines entsprechenden Imidazoliumchlorids in Dichlormethan, wobei mit einer wässrigen Lösung von konzentrierter Schwefelsäure umgesetzt wird und die HCl-haltigen Nebenprodukte abdestilliert werden.

WO 03/002579 beschreibt die Umsetzung der Trifluortris(pentafluorethyl)-phosphorsäure mit einer wässrigen Lösung von Tetra(n-butyl)phosphoniumchlorid in dem Lösungsmittelgemisch Diethylether/Toluol und anschließender Abtrennung des Lösungsmittelgemischs durch Destillation.

In David James H., et al, "Synthesis and purification of ionic liquids" 2003, Ionic Liquids in Synthesis, 7-21, ed. Wasserscheid Peter, Welton Thomas, Wiley-VCH Verlag GmbH & Co. KGaA, Weinheim, Deutschland, ISBN 3-527-30515-7 werden Reinigungsmethoden für ionische Flüssigkeiten beschrieben, beispielsweise das Auswaschen der entstandenen Säure mit Wasser.

Alle bekannten Verfahren haben einen Nachteil, insbesondere für eine großtechnische Synthese, was durch das Beispiel der Synthese von ionischen Flüssigkeiten mit Tetrafluorborat-Anionen verdeutlicht werden kann. Silbertetrafluoroborat ist beispielsweise ein teures Reagenz. Die Umsetzungen mit NaBF₄, NH₄BF₄ und HBF₄ in Wasser erfordern einen Aufreinigungsschritt, eventuell durch Verwendung von AgBF₄ oder Adsorbentien. HBF₄ in Methanol ist nicht kommerziell erhältlich und ist teurer als wässrige HBF₄, die wiederum kommerziell erhältlich ist.

Das Reaktionsmedium der Wahl, hinsichtlich einer großtechnischen Synthese von Onium-Salzen, die wasserlöslich oder teilweise wasserlöslich sind, sind wasserlösliche Säuren, beispielsweise HBF₄, H₂SiF₆, H₂TiF₆, H₂ZrF₆, HSbF₆, HAsF₆, HPF₆, HN(CN)₂, HC(CN)₃, H₂SO₄, HNO₃, Alkyl- oder Perfluoralkylsulfonsäuren, aromatische Sulfonsäuren, Perfluoralkylcarbonsäuren, Alkyl- oder Perfluoralkylphosphinsäuren, Alkyl- oder Perfluoralkylphosphonsäuren, aromatische Phosphin- oder Phosphonsäuren oder Phosphorsäure. Es gibt bei der genannten Umsetzung, die vorteilhaft in Wasser oder auch in mit Wasser mischbaren Lösungsmittels durchgeführt wird, jedoch ein generelles Problem, da sich die bildende Halogenwasserstoffsäure nicht vollständig durch Destillation entfernen lässt. Es bildet sich bei Entfernen des Lösungsmittels immer ein Gleichgewicht zwischen zwei Salzen und zwei Säuren. Die erhaltenen Onium-Salze enthalten zwangsläufig immer einige Prozente an Halogenidionen, dokumentiert durch Untersuchungen von N.M.M Mateus et al, Green Chemistry, 5 (2003), 347-352.

Überraschenderweise wurde ein einfaches Verfahren entwickelt. Nach Umsetzung eines Onium-Chlorids mit einer Säure, gemäß Anspruch 1, wie zuvor als zweite Stufe der bekannten Reaktionen beschrieben, kann die entstehende Chlorwasserstoffsäure erfindungsgemäß durch Koordination an 1,4-Dioxan, welches ein azeotropes Gemisch mit Wasser bildet, durch azeotrope Destillation entfernt werden. Durch die azeotrope Destillation wird das oben beschriebene Gleichgewicht verschoben und man erhält nach mehrmaliger Destillation bei diskontinuierlicher oder halbkontinuierlicher Reaktionsführung oder durch kontinuierliche Destillation bei kontinuierlicher Reaktionsführung ionische Flüssigkeiten, deren Halogenid-Gehalt unter 5000 ppm (= 0,5 %), vorzugsweise unter 500 ppm, besonders bevorzugt unter 100 ppm, ganz besonders bevorzugt unter 20 ppm liegen kann.

Organische Lösungsmittel, die ein azeotropes Gemisch mit Wasser bilden können, sind beispielsweise Nitroalkane, Nitrile, Aromaten, cyclische oder lineare Ether oder Ester oder Alkohole. Ohne Einschränkung der Allgemeinheit sind Beispiele dieser Lösungsmittel 1,4-Dioxan, Ethanol, Propanol, Isopropanol, Butanol, Nitromethan, Acetonitril, Dimethoxyethan, Tetrahydrofuran, Isobuttersäurenitril, Cyclohexanon, Benzol oder Toluol.

Ganz besonders geeignet ist die Verwendung von 1,4-Dioxan.

Das erfindungsgemäße Verfahren kann zur Herstellung von Onium-Salzen verwendet werden, deren Kation beispielsweise Ammonium Phosphonium. Thiouronium, Guanidinium oder ein heterocyclisches Kation gemaß Anspruch 1 bedeutet und deren Anion das Anion der korrespondierenden Säure ist.

Geeignete Säuren sind, wie zuvor beschrieben HBF₄, H₂SiF₆, H₂TiF₆, H₂ZrF₆, HSbF₆, HAsF₆, HPF₆, HN(CN)₂, HC(CN)₃, H₂SO₄, HNO₃, Alkyl- oder Perfluoralkylsulfonsäuren, aromatische Sulfonsäuren, Perfluoralkylcarbonsäuren, Alkyl- oder Perfluoralkylphosphinsäuren, Alkyl- oder Perfluoralkylphosphonsäuren, aromatische Phosphin- oder Phosphonsäuren oder Phosphorsäure. Besonders geeignet ist das erfindungsgemäße Verfahren nach Anspruch 1 für die Umsetzung mit wässriger HBF₄, H₂SiF₆, H₂TiF₆, H₂SO₄, CF₃SO₃H, CF₃COOH, Toluolsulfonsäure-monohydrat oder CH₃SO₃H. Als Auswahl aus der besonders geeigneten Gruppe der Säuren, sind wiederum bevorzugt die Umsetzung mit wässriger HBF₄, H₂SO₄, CF₃SO₃H, H₂SiF₆ oder HpTiF₆. Das erfindungsgemäße Verfahren ist ganz besonders geeignet für die Umsetzung mit HBF₄ in Wasser.

Anionen der Onium-Salze sind demzufolge [BF₄]-, [SiF₆]²⁻, [TiF₆]²⁻, [SbF₆]⁻, [AsF₆]⁻, [PF₆]⁻, [N(GN)₂]⁻, [C(CN)₃]⁻, [HSO₄]⁻, [NO₃]⁻, Alkyl- oder Perfluoralkylsulfonat, beispielsweise [CH₃SO₃]⁻, [C₂H₅SO₃]⁻, [CF₃SO₃]⁻, [C₂F₅SO₃]⁻, Anionen aromatischer Sulfonsäuren, beispielsweise Tosylat, Mesylat oder Phenylsulfonat, Perfluoralkylcarboxylate, beispielsweise [CF3CO₂⁻] oder [C₂F₅CO₂]⁻, Anionen der Alkyl- oder
Perfluoralkylphosphinsäuren, beispielsweise (CH₃)₂P(O)O⁻, (C₂H₅)₂P(O)O⁻, (C₃H₇)₂P(O)O⁻, (C₄H₉)₂P(O)O⁻, (C₂F₅)₂P(O)O⁻, (C₃F₇)₂P(O)O⁻,
(C₄F₉)₂P(O)O⁻, Anionen der Alkyl- oder Perfluoralkylphosphonsäuren, beispielsweise [(C₂F₅)P(O)O₂]²⁻, [(C₃F₇)P(O)O₂]²⁻, [(C₄F₉)P(O)O₂]²-, Anionen aromatischer Phosphin- oder Phosphonsäuren, beispielsweise (C₆H₅)₂P(O)O⁻, oder Phosphate.

Geeignete Onium-Chloride sind Ammoniumchloride, Phosphoniumchloride, Thiouronium, Guanidiniumchloride oder Chloride mit heterocyclischem Kation, ausgewählt aus
Ammoniumchloriden der Formel (1)

[NR₄]⁺ Cl⁻ (1),

Phosphoniumchloriden der Formel (2)

[PR₄]⁺ Cl⁻ (2),

, wobei
R jeweils unabhängig voneinander
H, wobei nicht alle Substituenten R gleichzeitig H sein dürfen,
geradkettiges oder verzweigtes Alkyl mit 1-20 C-Atomen,
geradkettiges oder verzweigtes Alkenyl mit 2-20 C-Atomen und einer oder
mehreren Doppelbindungen,
geradkettiges oder verzweigtes Alkinyl mit 2-20 C-Atomen und einer oder
mehreren Dreifachbindungen,
gesättigtes, teilweise oder vollständig ungesättigtes Cycloalkyl mit 3-7 C-Atomen, das mit Alkylgruppen mit 1-6 C-Atomen substituiert sein kann,
wobei ein oder mehrere R teilweise oder vollständig mit Halogenen, insbesondere -F und/oder -Cl, oder teilweise mit -NO₂, substituiert sein können,
und wobei ein oder zwei nicht benachbarte und nicht α-ständige Kohlenstoffatome des R, durch Atome und/oder Atomgruppierungen ausgewählt aus der Gruppe -O-, -S-, -S(O)-, -SO₂- oder -P(O)R'- mit R' = nicht, teilweise oder perfluoriertes C₁- bis C₆-Alkyl, C₃- bis C₇-Cycloalkyl, unsubstituiertes oder substituiertes Phenyl, ersetzt sein können, Thiouroniumchloriden der Formel (3),

[(R¹R²N)-C(=SR⁷)(NR³R⁴)]⁺ Cl⁻ (3),

Guanidiniumchloriden der Formel (4)

[C(NR¹R²)(NR³R⁴)(NR⁵R⁶)]⁺ Cl⁻ (4),

, wobei
R¹ bis R⁷ jeweils unabhängig voneinander
Wasserstoff, wobei Wasserstoff für R⁷ ausgeschlossen wird, geradkettiges oder verzweigtes Alkyl mit 1 bis 20 C-Atomen, geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das mit Alkylgruppen mit 1-6 C-Atomen substituiert sein kann, bedeutet,
wobei ein oder mehrere der Substituenten R¹ bis R⁷ teilweise oder vollständig mit Halogenen, insbesondere -F und/oder -Cl, oder teilweise mit
-NO₂, substituiert sein können, wobei jedoch nicht alle Substituenten an einem N-Atom vollständig mit Halogenen substituiert sein dürfen,
und wobei ein oder zwei nicht benachbarte und nicht direkt am Heteroatom gebundene Kohlenstoffatome der Substituenten R¹ bis R⁶ durch Atome und/oder Atomgruppierungen ausgewählt aus der Gruppe -O-, -S-, -S(O)-,-SO₂- oder -P(O)R'- mit R' = nicht, teilweise oder perfluoriertes C₁- bis C₆-Alkyl, C₃- bis C₇-Cycloalkyl, unsubstituiertes oder substituiertes Phenyl, ersetzt sein können. oder
Chloriden mit heterocyclischem Kation der Formel (6)

[HetN]⁺ Cl⁻ (5)

wobei
HetN⁺ ein heterocyclisches Kation, ausgewählt aus der Gruppe 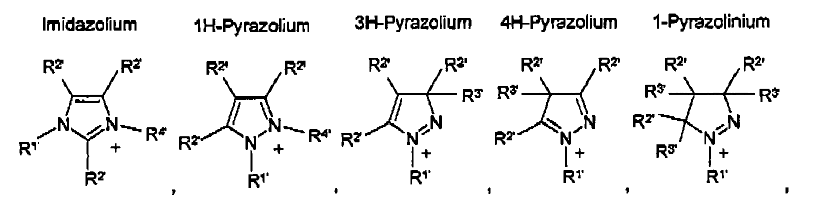 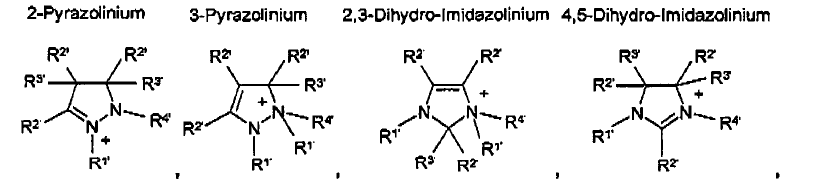 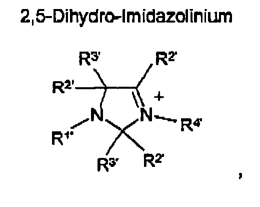 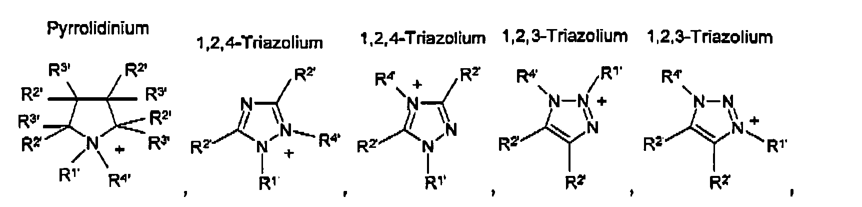 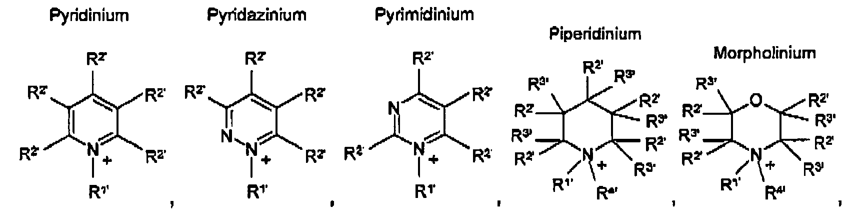 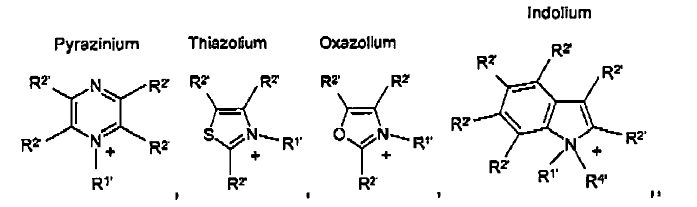 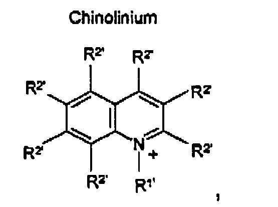 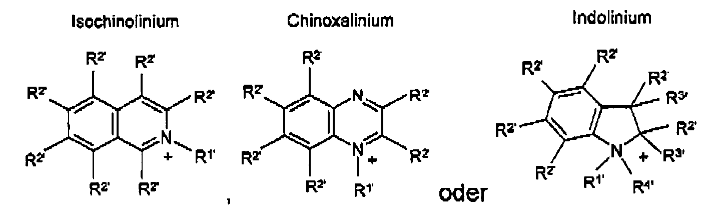 bedeutet, wobei die Substituenten
R^{1,} bis R⁴' jeweils unabhängig voneinander
Wasserstoff,
geradkettiges oder verzweigtes Alkyl mit 1-20 C-Atomen,
geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das mit Alkylgruppen mit 1-6 C-Atomen substituiert sein kann, gesättigtes, teilweise oder vollständig ungesättigtes Heteroaryl,
Heteroaryl-C₁-C₆-alkyl oder Aryl-C₁-C₆-alkyl bedeutet,
wobei die Substituenten R^{1'}, R^{2'}, R^{3'} und/oder R^{4'} zusammen auch ein Ringsystem bilden können,
wobei ein oder mehrere Substituenten R^{1,} bis R^{4,} teilweise oder vollständig mit Halogenen, insbesondere -F und/oder -Cl, oder teilweise mit -NO₂, substituiert sein können, wobei jedoch nicht gleichzeitig R^{1'} und R^{4'} vollständig mit Halogenen substituiert sein dürfen,
und wobei ein oder zwei nicht benachbarte und nicht am Heteroatom gebundene Kohlenstoffatome der Substituenten R^{1,} bis R⁴', durch Atome und/oder Atomgruppierungen ausgewählt aus der Gruppe -O-, -S-, -S(O)-, - SO₂- oder -P(O)R'- mit R' = nicht, teilweise oder perfluoriertes C₁- bis C₆-Alkyl, C₃- bis C₇-Cycloalkyl, unsubstituiertes oder substituiertes Phenyl, ersetzt sein können.

Ausgeschlossen sind jedoch Verbindungen der Formeln (1) und (2), in denen alle vier oder drei Substituenten R vollständig mit Halogenen substituiert sind, beispielsweise Tris(trifluormethyl)methylammoniumchlorid,
Tetra(trifluormethyl)ammoniumchlorid oder
Tetra(nonafluorbutyl)ammoniumchlorid.

Unter vollständig ungesättigten Substituenten werden im Sinne der vorliegenden Erfindung auch aromatische Substituenten verstanden.

Als Substituenten R und R¹ bis R⁷ der Verbindungen der Formeln (1) bis (5) kommen erfindungsgemäß dabei neben Wasserstoff bevorzugt in Frage: C₁- bis C₂₀-, insbesondere C₁- bis C₁₄-Alkylgruppen, und gesättigte oder ungesättigte, d.h. auch aromatische, C₃- bis C₇-Cycloalkylgruppen, die mit C₁- bis C₆-Alkylgruppen substituiert sein können, insbesondere Phenyl.

Die Substituenten R in den Verbindungen der Formel (1) oder (2) können dabei gleich oder verschieden sein. Bevorzugt sind die Substituenten R gleich.

Der Substituent R ist insbesondere bevorzugt Methyl, Ethyl, Isopropyl, Propyl, Butyl, sek.-Butyl, Pentyl, Hexyl, Octyl, Decyl oder Tetradecyl.

Bis zu vier Substituenten des Guanidinium-Kations
[C(NR¹R²)(NR³R⁴)(NR⁵R⁶)]⁺ können auch paarweise derart verbunden sein, dass mono-, bi- oder polycyclische Kationen entstehen.

Ohne Einschränkung der Allgemeinheit sind Beispiele für solche Guanidinium-Kationen: 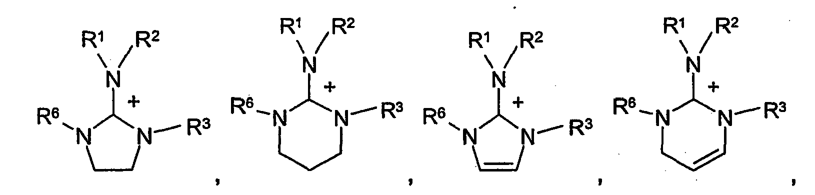 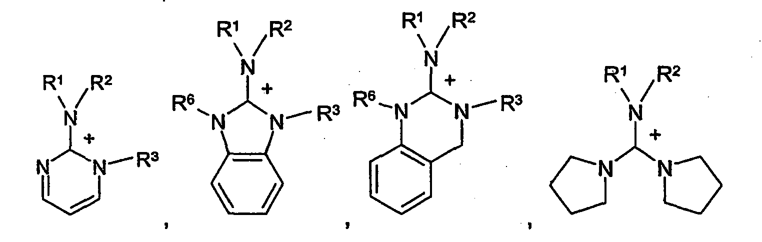 oder 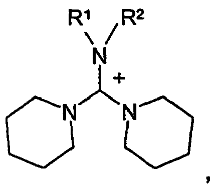 wobei die Substituenten R¹ bis R³ und R⁶ eine
zuvor angegebene oder besonders bevorzugte Bedeutung haben können. Gegebenenfalls können die Carbocyclen oder Heterocyclen der zuvor angegebenen Guanidinium-Kationen noch durch C₁- bis C₆-Alkyl, C₁- bis C₆-Alkenyl, NO₂, F, Cl, Br, I, OH, C₁-C₆-Alkoxy, SCF₃, SO₂CF₃, COOH, SO₂NR"₂, SO₂X' oder SO₃H substituiert sein, wobei X' und R" eine zuvor oder später angegebene Bedeutung haben, substituiertes oder unsubstituiertes Phenyl oder unsubstituierter oder-substituierter Heterocyclus substituiert sein.

Bis zu vier Substituenten des Thiouroniumkations [(R¹R²N)-C(=SR⁷)(NR³R⁴)]⁺ können auch paarweise derart verbunden sein, dass mono-, bi- oder polycyclische Kationen entstehen.

Ohne Einschränkung der Allgemeinheit sind Beispiele für solche Kationen im folgenden angegeben, wobei Y = S bedeutet: 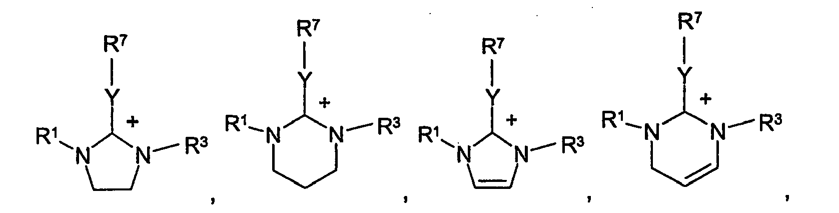 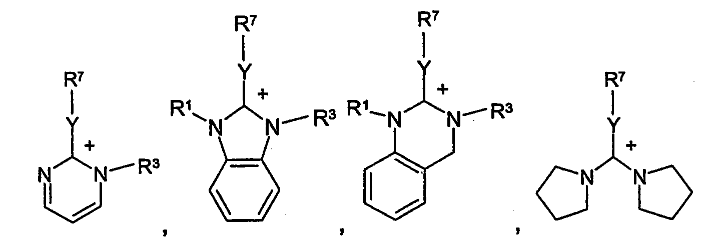 oder 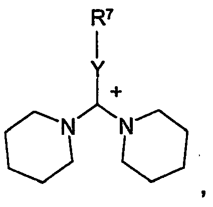 wobei die Substituenten R¹, R³ und R⁷ eine zuvor
angegebene oder besonders bevorzugte Bedeutung haben können. Gegebenenfalls können die Carbocyclen oder Heterocyclen der zuvor angegebenen Kationen noch durch C₁- bis C₆-Alkyl, C₁- bis C₆-Alkenyl, NO₂, F, Cl, Br, I, OH, C₁-C₆-Alkoxy, SCF₃, SO₂CF₃, COOH, SO₂NR"₂, SO₂X' oder SO₃H oder substituiertes oder unsubstituiertes Phenyl oder unsubstituierter oder substituierter Heterocyclus substituiert sein, wobei X' und R" eine zuvor angegebene Bedeutung haben.

Die Substituenten R¹ bis R⁷ sind jeweils unabhängig voneinander bevorzugt eine geradkettige oder verzweigte Alkylgruppe mit 1 bis 10 C-Atomen. Die Substituenten R¹ und R², R³ und R⁴ und R⁵ und R⁶ in Verbindungen der Formeln (3) bis (5) können dabei gleich oder verschieden sein. Besonders bevorzugt sind R¹ bis R⁷ jeweils unabhängig voneinander Methyl, Ethyl, n-Propyl, Isopropyl, n-Butyl, sek.-Butyl, Phenyl oder Cyclohexyl, ganz besonders bevorzugt Methyl, Ethyl, n-Propyl, Isopropyl oder n-Butyl.

Als Substituenten R^{1'} bis R^{4'} von Verbindungen der Formel (6) kommen erfindungsgemäß dabei neben Wasserstoff bevorzugt in Frage: C₁- bis C₂₀-, insbesondere C₁- bis C₁₂-Alkylgruppen, und gesättigte oder ungesättigte, d.h. auch aromatische, C₃- bis C₇-Cycloalkylgruppen, die mit C₁- bis C₆-Alkylgruppen substituiert sein können, insbesondere Phenyl.

Die Substituenten R^{1'} und R^{4'} sind jeweils unabhängig voneinander insbesondere bevorzugt Methyl, Ethyl, Isopropyl, Propyl, Butyl, sek.-Butyl, Pentyl, Hexyl, Octyl, Decyl, Cyclohexyl, Phenyl oder Benzyl. Sie sind ganz besonders bevorzugt Methyl, Ethyl, n-Butyl oder Hexyl. In Pyrrolidinium-, Piperidinium- oder Indolinium-Verbindungen sind die beiden Substituenten R^{1'} und R^{4'} bevorzugt unterschiedlich.

Der Substituent R^{2'} oder R^{3'} ist jeweils unabhänigig voneinander insbesondere Wasserstoff, Methyl, Ethyl, Isopropyl, Propyl, Butyl, sek.-Butyl, Cyclohexyl, Phenyl oder Benzyl. Besonders bevorzugt ist R^{2'} Wasserstoff, Methyl, Ethyl, Isopropyl, Propyl, Butyl oder sek.-Butyl. Ganz besonders bevorzugt sind R^{2'} und R^{3'} Wasserstoff.

Die C₁-C₁₂-Alkylgruppe ist beispielsweise Methyl, Ethyl, Isopropyl, Propyl, Butyl, sek.-Butyl oder tert.-Butyl, ferner auch Pentyl, 1-, 2- oder 3-Methylbutyl, 1,1-, 1,2-oder 2,2-Dimethylpropyl, 1-Ethylpropyl, Hexyl, Heptyl, Octyl, Nonyl, Decyl, Undecyl oder Dodecyl. Gegebenenfalls Difluormethyl, Trifluormethyl, Pentafluorethyl, Heptafluorpropyl oder Nonafluorbutyl.

Ein geradkettiges oder verzweigtes Alkenyl mit 2 bis 20 C-Atomen, wobei auch mehrere Doppelbindungen vorhanden sein können, ist beispielsweise Allyl, 2- oder 3-Butenyl, Isobutenyl, sek.-Butenyl, ferner 4-Pentenyl, iso-Pentenyl, Hexenyl, Heptenyl, Octenyl, -C₉H₁₇, -C₁₀H₁₉ bis -C₂₀H₃₉; vorzugsweise Allyl, 2- oder 3-Butenyl, Isobutenyl, sek.-Butenyl, ferner bevorzugt ist 4-Pentenyl, iso-Pentenyl oder Hexenyl.

Ein geradkettiges oder verzweigtes Alkinyl mit 2 bis 20 C-Atomen, wobei auch mehrere Dreifachbindungen vorhanden sein können, ist beispielsweise Ethinyl, 1- oder 2-Propinyl, 2- oder 3-Butinyl, ferner 4-Pentinyl, 3-Pentinyl, Hexinyl, Heptinyl, Octinyl, -C₉H₁₅, -C₁₀H₁₇ bis -C₂₀H₃₇, vorzugsweise Ethinyl, 1- oder 2-Propinyl, 2- oder 3-Butinyl, 4-Pentinyl, 3-Pentinyl oder Hexinyl.

Aryl-C₁-C₆-alkyl bedeutet beispielsweise Benzyl, Phenylethyl, Phenylpropyl, Phenylbutyl, Phenylpentyl oder Phenylhexyl, wobei sowohl der Phenylring als auch die Alkylenkette, wie zuvor beschrieben teilweise oder vollständig mit Halogenen, insbesondere -F und/oder -Cl, oder teilweise mit -NO₂, substituiert sein können.

Unsubstituierte gesättigte oder teilweise oder vollständig ungesättigte Cycloalkylgruppen mit 3-7 C-Atomen sind daher Cyclopropyl, Cyclobutyl, Cyclopentyl, Cyclohexyl, Cycloheptyl, Cyclopentenyl, Cyclopenta-1,3-dienyl, Cyclohexenyl, Cyclohexa-1,3-dienyl, Cyclohexa-1,4-dienyl, Phenyl, Cycloheptenyl, Cyclohepta-1,3-dienyl, Cyclohepta-1,4-dienyl oder Cyclohepta-1,5-dienyl, welche mit C₁- bis C₆-Alkylgruppen substituiert sein können, wobei wiederum die Cycloalkylgruppe oder die mit C₁- bis C₆-Alkylgruppen substituierte Cycloalkylgruppe auch mit Halogenatomen wie F, Cl, Br oder I, insbesondere F oder Cl oder NO₂ substituiert sein kann.

In den Substituenten R, R¹ bis R⁶ oder R^{1'} bis R^{4'} können auch ein oder zwei nicht benachbarte und nicht □-ständig zum Heteroatom gebundene Kohlenstoffatome, durch Atome und/oder Atomgruppierungen ausgewählt aus der Gruppe -O-, -S-, -S(O)-, -SO₂- oder -P(O)R'- ersetzt werden, mit R' = nicht, teilweise oder perfluoriertes C₁- bis C₆-Alkyl, C₃- bis C₇-Cycloalkyl, unsubstituiertes oder substituiertes Phenyl.

Ohne Einschränkung der Allgemeinheit sind Beispiele für derart modifizierte Substituenten R, R¹ bis R⁶ und R^{1'} bis R^{4'}:

-OCH₃, -OCH(CH₃)₂, -CH₂OCH₃, -CH₂-CH₂-O-CH₃, -C₂H₄OCH(CH₃)₂,C₂H₄SC₂H₅, -C₂H₄SCH(CH₃)₂, -S(O)CH₃, -SO₂CH₃, -SO₂C₆H₅, -SO₂C₃H₇,SO₂CH(CH₃)₂, -SO₂CH₂CF₃, -CH₂SO₂CH₃, -O-C₄H₈-O-C₄H₉, -CF₃, -C₂F₅,C₃F₇, -C₄F₉, -C(CF₃)₃, -CF₂SO₂CF₃, -C₂F₄N(C₂F₅)C₂F₅, -CHF₂, -CH₂CF₃,C₂F₂H₃, -C₃FH₆, -CH₂C₃F₇, -CH₂C(O)OH, -CH₂C₆H₅, -C(O)C₆H₅ oder P(O)(C₂H₅)₂.

In R' ist C₃- bis C₇-Cycloalkyl beispielweise Cyclopropyl, Cyclobutyl, Cyclopentyl, Cyclohexyl oder Cycloheptyl.

In R' bedeutet substituiertes Phenyl, durch C₁- bis C₆-Alkyl, C₁- bis C₆-Alkenyl, NO₂, F, Cl, Br, I, OH, C₁-C₆-Alkoxy, SCF₃, SO₂CF₃, COOH, SO₂X', SO₂NR"₂ oder SO₃H substituiertes Phenyl, wobei X' F, Cl oder Br und R" ein nicht, teilweise oder perfluoriertes C₁- bis C₆-Alkyl oder C₃- bis C₇-Cycloalkyl wie für R' definiert bedeutet, beispielsweise, o-, m- oder p-Methylphenyl, o-, m- oder p-Ethylphenyl, o-, m- oder p-Propylphenyl, o-, m- oder p-Isopropylphenyl, o-, m- oder p-tert.-Butylphenyl, o-, m- oder p-Nitrophenyl, o-, m- oder p-Hydroxyphenyl, o-, m- oder p-Methoxyphenyl, o-, m- oder p-Ethoxyphenyl, o-, m-, p-(Trifluormethyl)phenyl, o-, m-, p-(Trifluormethoxy)phenyl, o-, m-, p-(Trifluormethylsulfonyl)phenyl, o-, m- oder p-Fluorphenyl, o-, m- oder p-Chlorphenyl, o-, m- oder p-Bromphenyl, o-, m- oder p-lodphenyl, weiter bevorzugt 2,3-, 2,4-, 2,5-, 2,6-, 3,4- oder 3,5-Dimethylphenyl, 2,3-, 2,4-, 2,5-, 2,6-, 3,4- oder 3,5-Dihydroxyphenyl, 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,3-, 2,4-, 2,5-, 2,6-, 3,4- oder 3,5-Dimethoxyphenyl, 5-Fluor-2-methylphenyl, 3,4,5-Trimethoxyphenyl oder 2,4,5-Trimethylphenyl.

In R^{1'} bis R^{4'} wird als Heteroaryl ein gesättigter oder ungesättigter mono- oder bicyclischer heterocyclischer Rest mit 5 bis 13 Ringgliedern verstanden, wobei 1, 2 oder 3 N- und/oder 1 oder 2 S- oder O-Atome vorliegen können und der heterocyclische Rest ein- oder mehrfach durch C₁- bis C₆-Alkyl, C₁- bis C₆-Alkenyl, NO₂, F, Cl, Br, I, OH, C₁-C₆-Alkoxy, SCF₃, SO₂CF₃, COOH, SO₂X', SO₂NR"₂ oder SO₃H substituiert sein kann, wobei X' und R" eine zuvor angegebene Bedeutung haben.

Der heterocyclische Rest ist vorzugsweise substituiertes oder unsubstituiertes 2- oder 3-Furyl, 2- oder 3-Thienyl, 1-, 2- oder 3-Pyrrolyl, 1-, 2-, 4- oder 5-Imidazolyl, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -4- oder -5-yl, 1- oder 5-Tetrazolyl, 1,2,3-Oxadiazol-4- oder -5-yl 1,2,4-Oxadiazol-3-oder-5-yl, 1,3,4-Thiadiazol-2- oder -5-yl, 1,2,4-Thiadiazol-3- oder -5-yl, 1,2,3-Thiadiazol-4- oder -5-yl, 2-, 3-, 4-, 5- oder 6-2H-Thiopyranyl, 2-, 3- oder 4-4H-Thiopyranyl, 3- oder 4-Pyridazinyl, Pyrazinyl, 2-, 3-,4-,5-,6- oder 7-Benzofuryl, 2-, 3-, 4-, 5-, 6- oder 7-Benzothienyl, 1-, 2-, 3-,4-, 5-, 6-oder 7-1H-Indolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzthiazolyl, -2-, 4-, 5-, 6- oder 7-Benzisothiazolyl, 4-, 5-, 6- oder 7-Benz-2,1,3-oxadiazolyl, 1-, 2-, 3-, 4-, 5-, 6-, 7- oder 8-Chinolinyl, 1-, 3-, 4-, 5-, 6-, 7- oder 8-Isochinolinyl, 1-, 2-, 3-, 4- oder 9-Carbazolyl, 1-, 2-, 3-, 4-, 5-, 6-, 7-, 8- oder 9-Acridinyl, 3-, 4-, 5-, 6-, 7- oder 8-Cinnollnyl, 2-, 4-, 5-, 6-, 7- oder 8-Chinazolinyl oder 1-, 2- oder 3-Pyrrolidinyl.

Unter Heteroaryl-C₁-C₆-alkyl wird nun in Analogie zu Aryl-C₁-C₆-alkyl beispielsweise Pyridinyl-methyl, Pyridinyl-ethyl, Pyridinyl-propyl, Pyridinylbutyl, Pyridinyl-pentyl, Pyridinyl-hexyl verstanden, wobei weiterhin die zuvor beschriebenen Heterocyclen in dieser Weise mit der Alkylenkette verknüpft werden können.

HetN⁺ ist bevorzugt 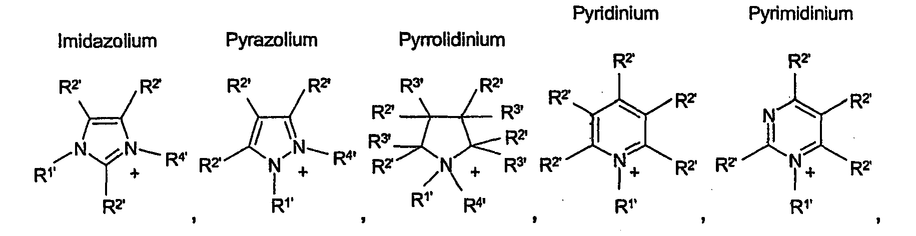 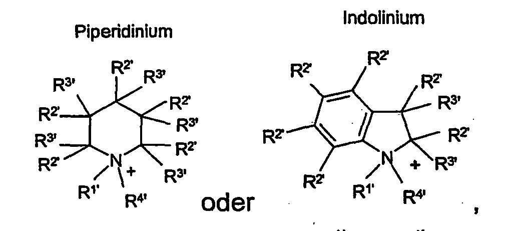 wobei die Substituenten R^{1'} bis R^{4'} jeweils unabhängig voneinander eine zuvor beschriebene Bedeutung haben.

HetN⁺ ist besonders bevorzugt Imidazolium, Pyrrolidinium oder Pyridinium, wie zuvor definiert, wobei die Substituenten R^{1'} bis R^{4'} jeweils unabhängig voneinander eine zuvor beschriebene Bedeutung haben. HetN⁺ ist ganz besonders bevorzugt Imidazolium, wobei die Substituenten R^{1'} bis R^{4'} jeweils unabhängig voneinander eine zuvor beschriebene Bedeutung haben.

Ein allgemeines Schema fasst das erfindungsgemäße Verfahren zusammen, wobei der Pfeil bei der entstehenden Chlorwasserstoffsäure HCl ein Symbol für die azeotrope Destillation darstellt:

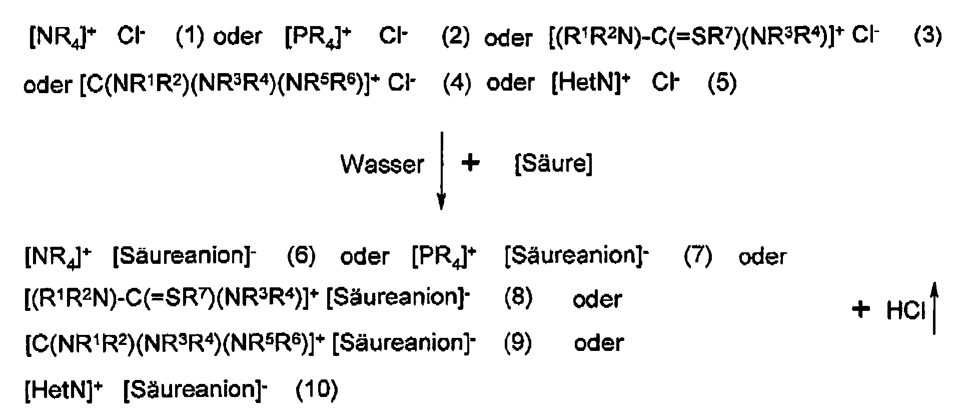

Die Substituenten R, R¹ bis R⁷ und HetN⁺ der Verbindungen der Formeln (1) bis (10) entsprechen den Bedeutungen, wie zuvor beschrieben.

Der Anionenaustausch wird unter Reaktionsbedingungen durchgeführt, die dem Fachmann bekannt sind, und es wird das Lösungsmittel Wasser verwendet.

Die Reaktion kann beispielsweise bei Temperaturen von 0°C bis 100°C, vorzugsweise bei 10° bis 50 °C, besonders bevorzugt bei Raumtemperatur durchgeführt werden. Die Umsetzung wird mit einem Überschuss oder equimolarer Menge an Säure durchgeführt. Vorzugsweise wird mit einem Überschuss von 0,1 bis 5 mol% an Säure gearbeitet. Die azeotrope Destillation wird unter Normaldruck oder unter reduziertem Druck mehrere Male durchgeführt. Unter reduziertem Druck sind dabei vorzugsweise Drücke von 0.1 Pa bis Normaldruck zu verstehen. Bevorzugt wird der niedrige Chloridgehalt erzielt, wenn die azeotrope Destillation fünf Mal durchgeführt wird. Besonders bevorzugt wird der niedrige Chloridgehalt bei dreimaliger Ausführung der azeotropen Destillation erreicht.

Auch ohne weitere Ausführungen wird davon ausgegangen, dass ein Fachmann die obige Beschreibung im weitesten Umfang nutzen kann. Die bevorzugten Ausführungsformen und Beispiele sind deswegen lediglich als beschreibende, keineswegs als in irgendeiner Weise limitierende Offenbarung aufzufassen.

Die NMR-Spektren wurden an Lösungen in deuterierten Lösungsmitteln bei 20°C an einem Bruker Avance 300 Spektrometer mit einem 5 mm Breitbandkopf ¹H/BB mit Deuterium Lock gemessen, falls nicht in den Beispielen angegeben. Die Messfrequenzen der verschiedenen Kerne sind: ¹H: 300,13 MHz, ¹¹B: 96,92 MHz, ¹⁹F: 282,41 MHz und ³¹P: 121,49 MHz. Die Methode der Referenzierung wird bei jedem Spektrum bzw. bei jedem Datensatz separat angegeben.

### Beispiel 1:

### Synthese von 1-Butyl-3-methylimidazolium tetrafluoroborat

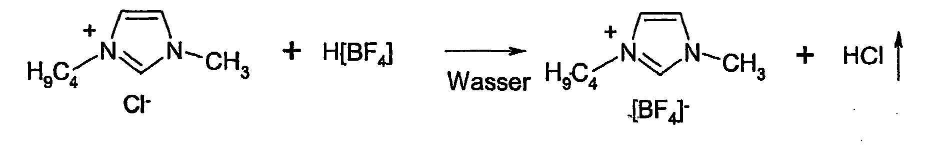

116,5 g (0,667 mol) 1-Butyl-3-methylimidazolium-chlorid werden im flüssigen Zustand bei 70 °C vorgelegt und in 120,6 g ca. 50 %-iger wässriger HBF₄ (ca. 3 % Überschuss) gelöst. Dabei wird keine Erwärmung und nur eine leichte HCl-Gasbildung beobachtet. Anschließend werden 250 ml 1,4-Dioxan zugegeben und bei Normaldruck 250 ml HCl-haltiges Wasser-Dioxan-Azeotrop abdestilliert (85-90 °C). Dann werden erneut 200 ml Dioxan zugegeben und bei Normaldruck 250 ml HCl-haltiges Wasser-Dioxan-Gemisch abdestilliert (85-101 °C). Der Rückstand im Destillationskolben weist einen geringen Gehalt an Chloridionen auf (Silbernitrattest). Nach erneuter azeotroper Destillation mit 100 ml Dioxan ist der Chloridionengehalt im Rückstand zu gering um mit dem Silbernitratest nachgewiesen zu werden.

Nach Trocknen des Destillationsrückstands unter reduziertem Druck bei 1.3 Pa und 80° C erhält man 149,2 g 1-Butyl-3-methylimidazolium-tetrafluoroborat als Flüssigkeit. Die Ausbeute an 1-Butyl-3-methylimidazollum-tetrafluoroborat ist annähernd quantitativ. Der Chloridgehalt in der ionischen Flüssigkeit beträgt 11 ppm, gemessen durch Mikrotitration mit einer Silbernitrat-Lösung im nichtwässrigen Milieu. Die Detektion des Endpunkts erfolgt potentiometrisch mit einer Cl-selektiven Elektrode.

¹H NMR Spektrum, ppm (Acetonitril-D₃ ; Referenz: TMS): 0.93 t (CH₃); 1.32 m (CH₂); 1.82 m (CH₂); 3.85 s (CH₃); 4.16 t (CH₂); 7.40 d,d (CH); 7.44 d,d (CH); 8.54 m (CH); ³J_{H,H} = 7.3 Hz; J_{H,H} = 1.8 Hz.

¹¹B NMR Spektrum, ppm (Acetonitril-D₃; Referenz: BF₃ - OEt₂ - extern): - 1.12 s (BF4⁻).

¹⁹F NMR Spektrum, ppm (Acetonitril-D₃; Referenz: CCl₃F - intern): -149.53 br.s (BF₄⁻).

### Beispiel 2:

### Synthese von 1-Butyl-4-methylpyridinium tetrafluoroborat

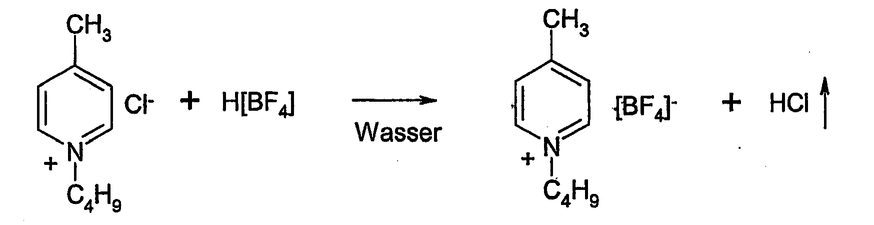

Zu 19,8 g (0,107 mol) 1-Butyl-4-methylpyridinium Chlorid werden 19,5 g einer 50%igen wässrigen Lösung von HBF₄ (0,111 mol) zugegeben und es wird 20 min bei Raumtemperatur gerührt. Danach werden 20 ml 1,4-Dioxan zugegeben und 28 ml azeotropes 1,4-Dioxan-Wasser-Gemisch bei Atmosphärendruck und 85-95°C abdestilliert. Der Vorgang wird wiederholt, bis kein Chlorid mehr im Silbernitrat-Test nachgewiesen werden kann. Nach Trocknen unter reduziertem Druck bei 1,3 Pa und 80°C, erhält man 25,1 g 1-Butyl-4-methylpyridinium tetrafluoroborat, das entspricht einer Ausbeute von 99,0 %, bezogen auf 1-Butyl-4-methylpyridinium Chlorid.

¹H NMR Spektrum, ppm (Acetonitril-D₃; Referenz: TMS): 0.93 t(CH₃); 1.35 m (CH₂); 1.93 m (CH₂); 2.63 s (CH₃); 4.50 t (CH₂); 7.86 d (2CH); 8.61 d (2CH); ³J_{H,H} = 7.4 Hz; ³J_{H,H} = 6.8 Hz.

¹¹B NMR Spektrum, ppm (Acetonitril-D₃; Referenz: BF₃ · OEt₂ - extern): - 1.11 s (BF₄⁻).

¹⁹F NMR Spektrum, ppm (Acetonitril-D₃; Referenz: CCl₃F - intern): -149.66 br.s (BF₄⁻).

### Beispiel 3:

### Synthese von 1-Ethtyl-3-methylimidazolium tetrafluoroborat

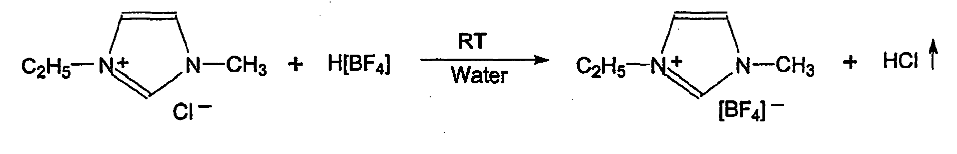

105 g (0,716 mol) 1-Ethyl-3-methylimidazolium Chlorid werden bei RT (Raumtemperatur) in 129,5 g ca. 50 %-iger wässriger HBF₄ (ca. 3 % Überschuss) gelöst. Dabei wird keine Erwärmung und nur eine leichte HCl-Gasbildung beobachtet. Anschließend werden 260 ml 1,4-Dioxan zugegeben und bei Normaldruck 260 ml HCl-haltiges Wasser-Dioxan-Azeotrop abdestilliert (85-90° C). Dann werden erneut 210 ml Dioxan zugegeben und bei Normaldruck 260 ml HCl-haltiges Wasser-Dioxan-Gemisch abdestilliert (85-101° C). Der Rückstand im Destillationskolben weist einen geringen Gehalt an Chloridionen auf (Silbernitrattest). Nach erneuter azeotroper Destillation mit 120 ml Dioxan ist der Chloridionengehalt im Rückstand zu gering um mit dem Silbernitratest nachgewiesen zu werden.

Nach Trocknen des Destillationsrückstands unter reduziertem Druck bei 1.3 Pa und 70° C wird 1-Ethyl-3-methylimidazolium-tetrafluoroborat als Flüssigkeit erhalten. Die Ausbeute ist annähernd quantitativ. Der Chloridgehalt in der ionischen Flüssigkeit beträgt weniger als 5 ppm, gemessen wie in Beispiel 1 beschrieben.

¹H NMR Spektrum, ppm (Acetonitril-D₃; Referenz: TMS): 1.44 t (CH₃); 3.85 s (CH₃); 4.18 q (CH₂); 7.40 d,d (CH); 7.46 d,d (CH); 8.53 m (CH); ³J_{H,H} = 7.3 Hz; J_{H,H} = 1.8 Hz.

¹⁹F NMR Spektrum, ppm (Acetonitril-D₃; Referenz: CCl₃F - intern): -149.12 br.s (BF₄⁻).

### Beispiel 4:

### Synthese von Trihexyl(tetradecyl)phosphonium Tetrafluoroborat

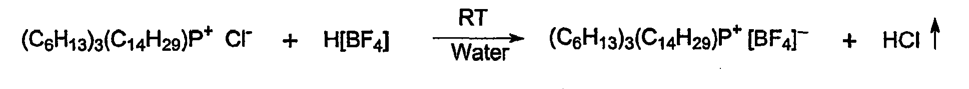

Analog zu Beispiel 1 werden 54,35 g (0,105 mol)

Trihexyl(tetradecyl)phosphonium Chlorid mit 18,90 g ca. 50 %-iger wässriger HBF₄ umgesetzt. Anschließend werden 40 ml 1,4-Dioxan zugegeben und bei Normaldruck 48 ml HCl-haltiges Wasser-Dioxan-Azeotrop abdestilliert (85-90° C). Dann werden erneut 30 ml Dioxan zugegeben und bei Normaldruck 30 ml HCl-haltiges Wasser-Dioxan-Gemisch abdestilliert (85-101° C). Nach erneuter azeotroper Destillation mit 30 ml Dioxan ist der Chloridionengehalt im Rückstand zu gering um mit dem Silbernitratest nachgewiesen zu werden.

Nach Trocknen des Destillationsrückstands unter reduziertem Druck bei 1.3 Pa und 70° C wird Trihexyl(tetradecyl)phosphonium Tetrafluoroborat als Flüssigkeit erhalten. Die Ausbeute ist annähernd quantitativ. Der Chloridgehalt in der ionischen Flüssigkeit beträgt 12 ppm, gemessen wie in Beispiel 1 beschrieben.

¹H NMR Spektrum, ppm (Acetonitril-D₃; Referenz: TMS): 0.91 t (CH₃); 0.93 t (3CH₃); 1,28-1,40 m (16CH₂); 1.40-1.62 m (8CH₂); 2.07-2.20 m (4CH₂); ³J_{H,H} = 7 Hz.

¹¹B NMR Spektrum, ppm (Acetonitril-D₃; Referenz: BF₃ · OEt₂ - extern):-1.22 s (BF₄⁻).

¹⁹F NMR Spektrum, ppm (Acetonitril-D₃; Referenz: CCt₃F - intern): -149.22 br.s (BF₄⁻).

### Beispiel 5: :

### Synthese von Tetrabutylammonium Tetrafluoroborat

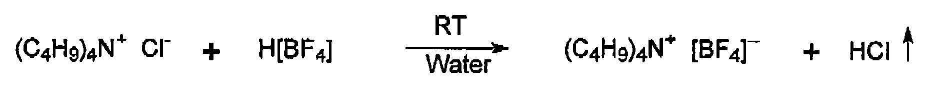

Analog zu Beispiel 1 werden 24,80 g (0,089 mol) Tetrabutylammonium Chlorid mit 16,2 g ca. 50 %-iger wässriger HBF₄ (ca. 3 % Überschuss) umgesetzt. Nach dreimaliger Zugabe von 40 ml 1,4-Dioxan und azeotroper Destillation von ca. 42 bis 45 ml HCl-haltigem Wasser-Dioxan-Azeotrop abdestilliert (85-90° C) ist der Chloridionengehalt im Rückstand im Silbernitrattest gering.

Nach Trocknen des Destillationsrückstands unter reduziertem Druck bei 1.3 Pa und 70° C werden 28,8 g Tetrabutylammonium Tetrafluoroborat erhalten. Die Ausbeute ist annähernd quantitativ. Der Chloridgehalt in der ionischen Flüssigkeit beträgt 118 ppm, gemessen wie in Beispiel 1 beschrieben.

¹H NMR Spektrum, ppm (Acetonitril-D₃; Referenz: TMS): 0.99 t (4CH₃); 1.38 t,q (4CH₂); 1.63 m (4CH₂); 3.11 s (4CH₂); 3.J_{H,H} = 7.2 Hz.

¹¹B NMR Spektrum, ppm (Acetonitril-D₃; Referenz: BF₃ · OEt₂ - extern):-1.24 s (BF₄⁻).

¹⁹F NMR Spektrum, ppm (Acetonitril-D₃; Referenz: CCl₃F - intern): -150.47 s (BF₄⁻).

### Beispiel 6: nicht gemäß Erfindung

### Synthese von 1-Butyl-3-methylimidazolium Trifluormethansulfonat

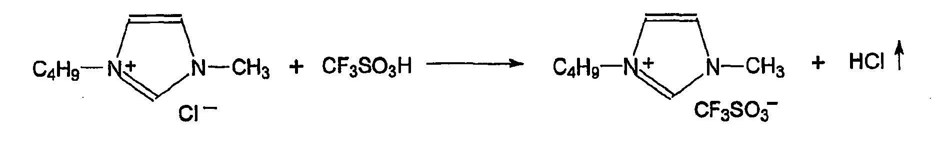

Analog zu Beispiel 1 werden 174,7 g (1,0 mol) 1-Butyl-3-methylimidazolium Chlorid mit 153,2 g (1,0 mol) 98 %-iger CF₃SO₃H umgesetzt. Anschließend werden 200 ml 1,4-Dioxan zugegeben und bei Normaldruck 200 ml HCl-haltiges Wasser-Dioxan-Azeotrop abdestilliert (85-101°C). Die azeotrope Destillation wird noch zwei Mal durchgeführt (Zugabe von je 150 ml Dioxan und abdestillieren von je 150 ml HCl-haltiges Wasser-Dioxan-Gemisch (95-101°C)).

Nach Trocknen des Destillationsrückstands unter reduziertem Druck bei 1.3 Pa und 70° C wird 1-Butyl-3-methylimidazolium Trifluormethansulfonat als Flüssigkeit erhalten. Die Ausbeute ist annähernd quantitativ. Der Chloridgehalt in der ionischen Flüssigkeit beträgt 9 ppm, gemessen wie in Beispiel 1 beschrieben.

¹H NMR Spektrum, ppm (Acetonitril-D₃; Referenz: TMS): 0.91 t (CH₃); 1.31 m (CH₂); 1.82 m (CH₂); 3.87 s (CH₃); 4.17 t (CH₂); 7.46 d,d (CH); 7.52 d,d (CH); 8.74 br.s (CH); ³J_{H,H} = 7.3 Hz; J_{H},_{H} = 1.8 Hz.

¹⁹F NMR Spektrum, ppm (Acetonitril-D₃; Referenz: CCl₃F - intern): -78.10 q,q (CF₃SO⁻₃).

### Beispiel 7:

### Synthese von 1-Hexyl-3-methylimidazolium Hexafluorsilikat

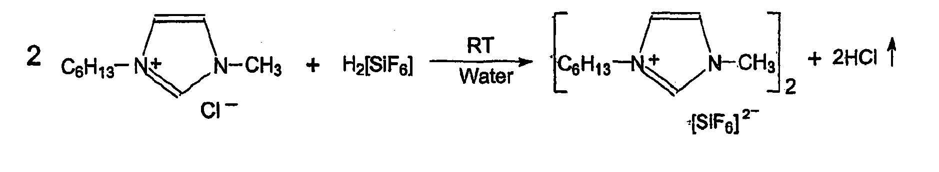

Analog zu Beispiel 1 werden 150,1 g (0,740 mol) 1-Hexyl-3-methylimidazolium-chlorid mit 218,8 g ca. 25 %-iger wässriger H₂SiF₆ (ca. 3 % Überschuss) umgesetzt. Anschließend werden 600 ml 1,4-Dioxan zugegeben und bei Normaldruck 695 ml HCl-haltiges Wasser-Dioxan-Azeotrop abdestilliert (85°C). Dann werden erneut 310 ml Dioxan zugegeben und bei Normaldruck 380 ml HCl-haltiges Wasser-Dioxan-Gemisch abdestilliert (85-101 °C). Ein Silbernitrattest kann aufgrund der Schwerlöslichkeit von Silber(I)hexafluorosilikat nicht durchgeführt werden. Daraufhin wurde noch drei Mal mit je 100 ml Dioxan azeotrop destilliert. Nach Trocknen des Destillationsrückstands unter reduziertem Druck bei 1.3 Pa und 70° C erhält man 171,1 g 1-Hexyl-3-methylimidazolium Hexafluorsilikat als hochviskoses Produkt, das entspricht einer Ausbeute von 97,0 %.

¹H NMR Spektrum, ppm (Acetonitril-D₃; Referenz: TMS): 0.84 m (CH₃); 1.27 m (3CH₂); 1.83 m (CH₂); 3.91 s (CH₃); 4.22 t (CH₂); 7.60 d,d (CH); 7.64 d,d (CH); 9.73 m (CH); ³J_{H,H} = 7.3 Hz; J_{H,H} = 1.7 Hz.

¹⁹F NMR Spektrum, ppm (Acetonitril-D₃; Referenz: CCl₃F - intern): -136.27 br.s (SiF₆²⁻).

### Beispiel 8:

### Synthese von 1-Butyl-3-methylimidazolium Hexafluortitanat

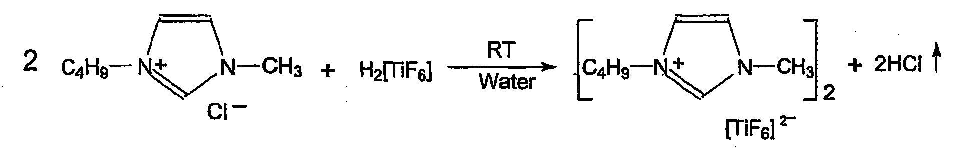

Analog zu Beispiel 1 werden 76,3 g (0,437 mol) 1-Butyl-3-methylimidazolium-chlorid mit 61,5 g ca. 60 %-iger wässriger H₂TiF₆ umgesetzt. Anschließend werden 100 ml 1,4-Dioxan zugegeben und bei Normaldruck 121 ml HCl-haltiges Wasser-Dioxan-Azeotrop abdestilliert (85°C). Dann werden erneut 100 ml Dioxan zugegeben und bei Normaldruck 104 ml HCl-haltiges Wasser-Dioxan-Gemisch abdestilliert (85-101 °C). Nach drei weiteren azeotropen Destillationen mit je 100 ml 1,4-Dioxan und Trocknung des Destillationsrückstands unter reduziertem Druck bei 1.3 Pa und 60° C wird 1-Butyl-3-methylimidazolium Hexafluortitanat als hochviskoses Produkt erhalten. Die Ausbeute ist annähernd quantitativ.

¹H NMR Spektrum, ppm (Acetonitril-D₃; Referenz: TMS): 0.88 t (CH₃); 1.28 m (CH₂); 1.77 m (CH₂); 3.86 s (CH₃); 4.17 t (CH₂); 7.42 d,d (CH); 7.43 d,d (CH); 8.54 m (CH); ³J_{H,H} = 7.3 Hz; J_{H,H} = 1.8 Hz.

### Beispiel 9:

### Synthese von 1-Butyl-3-methylimidazolium Tosylat

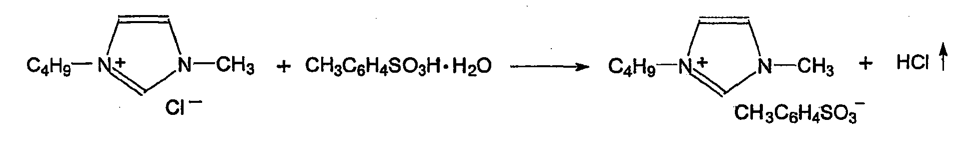

86,3 g (0,494 mol) 1-Butyl-3-methylimidazolium Chlorid und 94.9 g (0.499 mol) Toluolsulfonsäure-monohydrat werden bei RT in 50 ml 1,4-Dioxan gelöst. Anschließend werden bei Normaldruck 58 ml HCl-haltiges Wasser-Dioxan-Azeotrop abdestilliert (85-101°C). Nach 22 zusätzlichen azeotropen Destillationen mit je 200 ml Dioxan und Trocknung des Destillationsrückstands unter reduziertem Druck bei 1.3 Pa und 80° C wird 1-Butyl-3-methylimidazolium Tosylat erhalten. Die Ausbeute ist annähernd quantitativ.

¹H NMR Spektrum, ppm (Acetonitril-D₃; Referenz: TMS): 0.88 t (CH₃); 1.26 m (CH₂); 1.74 m (CH₂); 2.34 s (CH₃); 3.83 s (CH₃); 4.11 t (CH₂); 7.18 d,m (2 CH, A); 7.43 d,d (CH); 7.46 d,d (CH); 7.64 d,m (2 CH, B); 9.01 m (CH); ³J_{H,H} = 7.3 Hz; J_{A,B} = 8 Hz; J_{H,H} = 1.8 Hz.

### Beispiel 10:

### Synthese von 1-Butyl-3-methylimidazolium Methylsulfonat

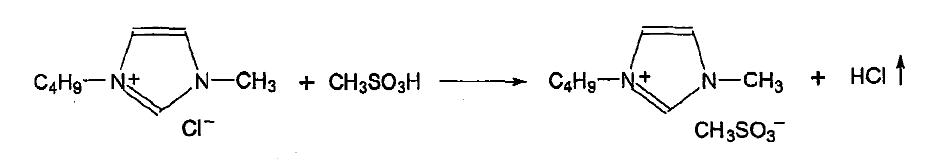

Analog zu Beispiel 1 werden 26,5 g (0,152 mol) 1-Butyl-3-methylimidazolium Chlorid mit 21.4 g ca. 70 %-iger wässriger Methansulfonsäure, CH₃SO₃H umgesetzt. Anschließend werden 100 ml 1,4-Dioxan zugegeben und bei Normaldruck 107 ml HCl-haltiges Wasser-Dioxan-Azeotrop abdestilliert (85-101 °C). Nach 18 zusätzlichen azeotropen Destillationen mit je 100 ml 1,4-Dioxan und Trocknung des Destillationsrückstands unter reduziertem Druck bei 1.3 Pa und 80° C wird 1-Butyl-3-methylimidazolium Methylsulfonat erhalten. Die Ausbeute ist annähernd quantitativ.

¹H NMR Spektrum, ppm (Acetonitril-D₃; Referenz: TMS): 0.89 t (CH₃); 1.28 m (CH₂); 1.80 m (CH₂); 2.60 s (CH₃); 3.89 s (CH₃); 4.20 t (CH₂); 7.57 d,d (CH); 7.61 d,d (CH); 9.34 m (CH); ³J_{H},_{H} = 7.3 Hz; J_{H,H} = 1.8 Hz.

### Beispiel 11:

### Synthese von 1-Butyl-3-methylimidazolium Hydrosulfat

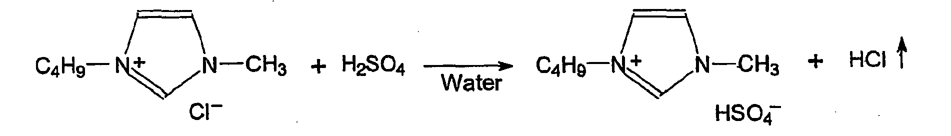

Analog zu Beispiel 1 werden 67,6 g (0,387 mol) 1-Butyl-3-methylimidazolium Chlorid mit 43.0 g ca. 90 %-iger wässriger Schwefelsäure, H₂SO₄, umgesetzt. Anschließend werden 80 ml 1,4-Dioxan zugegeben und bei Normaldruck 84 ml HCl-haltiges Wasser-Dioxan-Azeotrop abdestilliert (85-101 °C). Nach vier zusätzlichen azeotropen Destillationen mit je 80 ml 1,4-Dioxan und Trocknung des Destillationsrückstands unter reduziertem Druck bei 1.3 Pa und 80° C erhält man 1-Butyl-3-methylimidazolium Hydrosulfat. Die Ausbeute ist annähernd quantitativ.

¹H NMR Spektrum, ppm (Acetonitril-D₃; Referenz: TMS): 0.86 t (CH₃); 1.26 m (CH₂); 1.77 m (CH₂); 3.86 s (CH₃); 4.16 t (CH₂); 7.45 d,d (CH); 7.47 d,d (CH); 8.92 m (CH); 10.95 br.s (SO₄H); ³J_{H,H} = 7.3 Hz; J_{H,H} = 1.8 Hz.

### Beispiel 12:

### Synthese von 1-Butyl-3-methylimidazolium Trifluoracetat

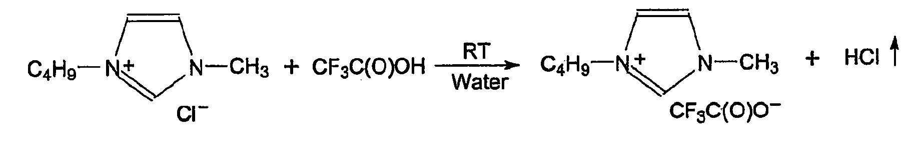

Analog zu Beispiel 1 werden 23,0 g (0,132 mol) 1-Butyl-3-methylimidazolium Chlorid mit 28.0 g ca. 80 %-iger wässriger Trifluoressigsäure, CF₃C(O)OH (50 % Überschuss) umgesetzt. Anschließend werden 100 ml 1,4-Dioxan zugegeben und bei Normaldruck 112 ml HCl- und CF₃C(O)OH-haltiges Wasser-Dioxan-Azeotrop abdestilliert (85-101 °C). Nach vier weiteren azeotropen Destillationen mit je 100 ml 1,4-Dioxan werden weitere 19 g ca. 80 %-iger wässriger Trifluoressigsäure zum Rückstand dazugegeben. Anschließend werden 100 ml 1,4-Dioxan zugegeben und bei Normaldruck 105 ml HCl- und CF₃C(O)OH-haltiges Wasser-Dioxan-Azeotrop abdestilliert. Nach drei weiteren azeotropen Destillationen mit je 100 ml 1,4-Dioxan und Trocknung des Destillationsrückstands unter reduziertem Druck bei 1.3 Pa und 70° C erhält man 1-Butyl-3-methylimidazolium Trifluoracetat. Die Ausbeute ist annähernd quantitativ. ¹H NMR Spektrum, ppm (Acetonitril-D₃; Referenz: TMS): 0.92 t (CH₃); 1.32 m (CH₂); 1.81 m (CH₂); 3.85 s (CH₃); 4.15 t (CH₂); 7.38 m (CH); 7.42 m (CH); 8.61 m (CH); ³J_{H,H} = 7.4 Hz. ¹⁹F NMR Spektrum, ppm (Acetonitril-D₃; Referenz:: CCl₃F - intern):

-75.60 s (CF₃C(O)O⁻).

## Patentansprüche

1. Verfahren zur Herstellung von Onium-Salzen mit geringem Chlorid-Gehalt durch Umsetzung eines Onium-Chlorids der Formel (1),
[NR₄]⁺ Cl⁻ (1),
oder der Formel (2),
[PR₄]⁺ Cl⁻ (2),
wobei
R jeweils unabhängig voneinander
H, wobei nicht alle Substituenten R gleichzeitig H sein dürfen, geradkettiges oder verzweigtes Alkyl mit 1-20 C-Atomen, geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das mit Alkylgruppen mit 1-6 C-Atomen substituiert sein kann, wobei ein oder mehrere R teilweise oder vollständig mit Halogenen, insbesondere -F und/oder -Cl, oder teilweise mit -NO₂, substituiert sein können,
wobei jedoch nicht alle vier oder drei R vollständig mit Halogenen substituiert sein dürfen,
und wobei ein oder zwei nicht benachbarte und nicht α-ständige Kohlenstoffatome des R, durch Atome und/oder Atomgruppierungen ausgewählt aus der Gruppe -O-, -S-, -S(O)-, -SO₂- oder -P(O)R'- mit R' = nicht, teilweise oder perfluoriertes C₁- bis C₆-Alkyl, C₃- bis C₇-Cycloalkyl, unsubstituiertes oder substituiertes Phenyl ersetzt sein können
oder der Formel (3),
[(R¹R²N)-C(=SR⁷)(NR³R⁴)]⁺ Cl⁻ (3),
wobei
R¹ bis R⁷ jeweils unabhängig voneinander
Wasserstoff, wobei Wasserstoff für R⁷ ausgeschlossen wird, geradkettiges oder verzweigtes Alkyl mit 1 bis 20 C-Atomen, geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das mit Alkylgruppen mit 1-6 C-Atomen substituiert sein kann, bedeutet,
wobei ein oder mehrere der Substituenten R¹ bis R⁷ teilweise oder vollständig mit Halogenen, insbesondere -F und/oder -Cl, oder teilweise mit -NO₂, substituiert sein können, wobei jedoch nicht alle Substituenten an einem N-Atom vollständig mit Halogenen substituiert sein dürfen, und wobei ein oder zwei nicht benachbarte und nicht direkt am Heteroatom gebundene Kohlenstoffatome der Substituenten R¹ bis R⁶ durch Atome und/oder Atomgruppierungen ausgewählt aus der Gruppe -O-, -S-, -S(O)-, -SO₂- oder -P(O)R'- mit R' = nicht, teilweise oder perfluoriertes C₁- bis C₆-Alkyl, C₃- bis C₇-Cycloalkyl, unsubstituiertes oder substituiertes Phenyl, ersetzt sein können
oder der Formel (4),
[C(NR¹R²)(NR³R⁴)(NR⁵R⁶)]⁺ Cl⁻ (4),
wobei
R¹ bis R⁶ jeweils unabhängig voneinander
Wasserstoff,
geradkettiges oder verzweigtes Alkyl mit 1 bis 20 C-Atomen, geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das mit Alkylgruppen mit 1-6 C-Atomen substituiert sein kann, bedeutet,
wobei ein oder mehrere der Substituenten R¹ bis R⁵ teilweise oder vollständig mit Halogenen, insbesondere -F und/oder -Cl, oder teilweise mit -NO₂, substituiert sein können, wobei jedoch nicht alle Substituenten an einem N-Atom vollständig mit Halogenen substituiert sein dürfen, und wobei ein oder zwei nicht benachbarte und nicht direkt am Heteroatom gebundene Kohlenstoffatome der Substituenten R¹ bis R⁶ durch Atome und/oder Atomgruppierungen ausgewählt aus der Gruppe-O-, -S-, -S(O)-, -SO₂- oder -P(O)R'- mit R' = nicht, teilweise oder perfluoriertes C₁- bis C₆-Alkyl, C₃- bis C₇-Cycloalkyl, unsubstituiertes oder substituiertes Phenyl, ersetzt sein können
oder der Formel (5),
[HetN]⁺ Cl⁻ (5)
wobei
HetN⁺ ein heterocyclisches Kation, ausgewählt aus der Gruppe 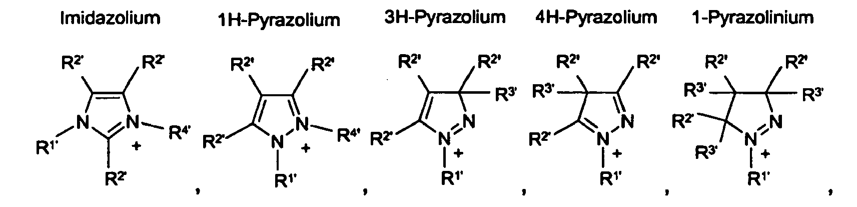 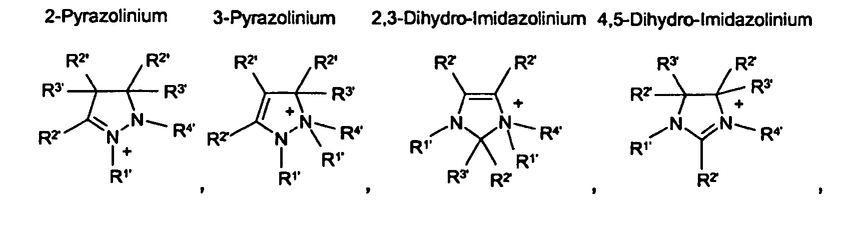 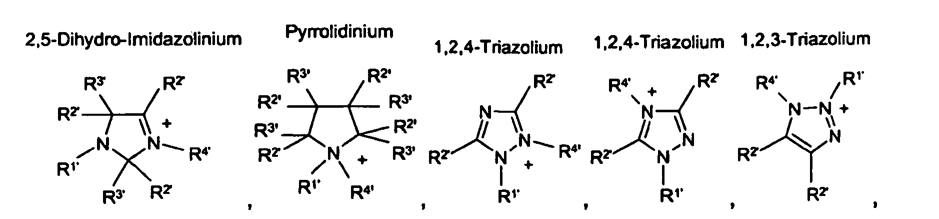 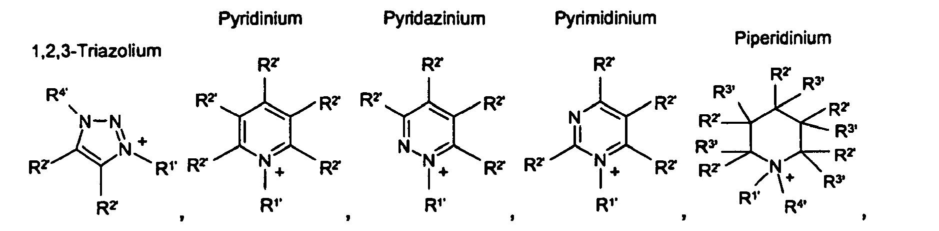 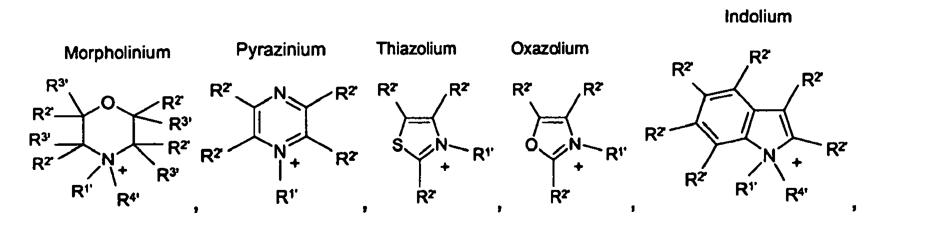 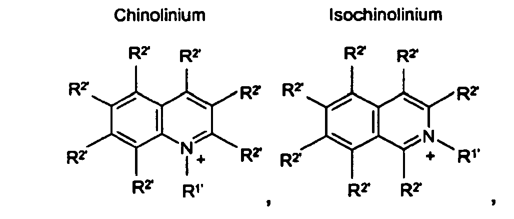 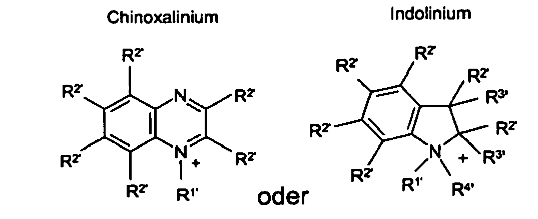 bedeutet, wobei die Substituenten
R¹' bis R^{4,} jeweils unabhängig voneinander
Wasserstoff,
geradkettiges oder verzweigtes Alkyl mit 1-20 C-Atomen, geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das mit Alkylgruppen mit 1-6 C-Atomen substituiert sein kann, gesättigtes, teilweise oder vollständig ungesättigtes Heteroaryl, Heteroaryl-C₁-C₆-alkyl oder Aryl-C₁-C₆-alkyl bedeutet,
wobei die Substituenten R^{1'}, R^{2'}, R^{3'} und/oder R^{4'} zusammen auch ein Ringsystem bilden können,
wobei ein oder mehrere Substituenten R^{1,} bis R^{4,} teilweise oder vollständig mit Halogenen, insbesondere -F und/oder -Cl, oder teilweise mit -NO₂, substituiert sein können, wobei jedoch nicht gleichzeitig R^{1'} und R^{4'} vollständig mit Halogenen substituiert sein dürfen,
und wobei ein oder zwei nicht benachbarte und nicht direkt am Heteroatom gebundene Kohlenstoffatome der Substituenten R^{1,} bis R^{4,}, durch Atome und/oder Atomgruppierungen ausgewählt aus der Gruppe-O-, -S-, -S(O)-, -SO₂- oder -P(O)R'- mit R' = nicht, teilweise oder perfluoriertes C₁- bis C₆-Alkyl, C₃- bis C₇-Cycloalkyl, unsubstituiertes oder substituiertes Phenyl, ersetzt sein können,
mit einer Säure, wobei die Reaktion in Wasser durchgeführt wird und die entstehende Chlorwasserstoffsäure durch Koordination an 1,4-Dioxan durch azeotrope Destillation entfernt wird.

2. Verfahren nach Anspruch 1, **dadurch gekennzeichnet, dass** eine Säure aus der Gruppe HBF₄, H₂SiF₆, H₂TiF₆, H₂ZrF₆, HSbF₆, HasF₆, HPF₆, HN(CN)₂, HC(CN)₃, H₂SO₄, HNO₃, Alkyl- oder Perfluoralkylsulfonsäuren, aromatische Sulfonsäuren, Perfluoralkylcarbonsäuren, Alkyl- oder Perfluoralkylphosphinsäuren, Alkyl- oder Perfluoralkylphosphonsäuren, aromatische Phosphin- oder Phosphonsäuren oder Phosphorsäure ausgewählt wird.

3. Verfahren nach Anspruch 1 oder 2, **dadurch gekennzeichnet, dass** die azeotrope Destillation diskontinuierlich, halbkontinuierlich oder kontinuierlich bei Normaldruck oder unter reduziertem Druck durchgeführt wird.

## Claims

1. Process for the preparation of onium salts having a low chloride content by reaction of an onium chloride of the formula (1)
[NR₄]⁺ Cl⁻ (1),
or of the formula (2)
[PR₄]⁺ Cl⁻ (2),
where
R in each case, independently of one another, denotes H, where all substituents R cannot simultaneously be H,
straight-chain or branched alkyl having 1-20 C atoms,
straight-chain or branched alkenyl having 2-20 C atoms and one or more double bonds,
straight-chain or branched alkynyl having 2-20 C atoms and one or more triple bonds,
saturated, partially or fully unsaturated cycloalkyl having 3-7 C atoms, which may be substituted by alkyl groups having 1-6 C atoms,
where one or more R may be partially or fully substituted by halogens, in particular -F and/or -Cl, or partially by -NO₂,
but where all four or three R cannot be fully substituted by halogens, and where one or two non-adjacent carbon atoms of the R which are not in the α-position may be replaced by atoms and/or atom groups selected from the group -O-, -S-, -S(O)-, -SO₂- or -P(O)R'-, where R' = non-, partially or perfluorinated C₁- to C₆-alkyl, C₃- to C₇-cycloalkyl, unsubstituted or substituted phenyl,
or of the formula (3)
[(R¹R²N)-C(=SR⁷)(NR³R⁴)]⁺ Cl⁻ (3),
where
R¹ to R⁷ each, independently of one another, denote hydrogen, where hydrogen is excluded for R⁷,
straight-chain or branched alkyl having 1 to 20 C atoms,
straight-chain or branched alkenyl having 2-20 C atoms and one or more double bonds,
straight-chain or branched alkynyl having 2-20 C atoms and one or more triple bonds,
saturated, partially or fully unsaturated cycloalkyl having 3-7 C atoms, which may be substituted by alkyl groups having 1-6 C atoms,
where one or more of the substituents R¹ to R⁷ may be partially or fully substituted by halogens, in particular -F and/or -Cl, or partially by -NO₂, but where all substituents on an N atom cannot be fully substituted by halogens,
and where, in the substituents R¹ to R⁶, one or two non-adjacent carbon atoms which are not bonded directly to the heteroatom may be replaced by atoms and/or atom groups selected from the group -O-, -S-, -S(O)-, -SO₂- or -P(O)R'-, where R' = non-, partially or perfluorinated C₁- to C₆-alkyl, C₃- to C₇-cycloalkyl, unsubstituted or substituted phenyl,
or of the formula (4)
[C(NR¹R²)(NR³R⁴)(NR⁵R⁶)]⁺ Cl⁻ (4),
where
R¹ to R⁶ each, independently of one another, denote hydrogen,
straight-chain or branched alkyl having 1 to 20 C atoms,
straight-chain or branched alkenyl having 2-20 C atoms and one or more double bonds,
straight-chain or branched alkynyl having 2-20 C atoms and one or more triple bonds,
saturated, partially or fully unsaturated cycloalkyl having 3-7 C atoms, which may be substituted by alkyl groups having 1-6 C atoms,
where one or more of the substituents R¹ to R⁶ may be partially or fully substituted by halogens, in particular -F and/or -Cl, or partially by -NO₂, but where all substituents on an N atom cannot be fully substituted by halogens,
and where, in the substituents R¹ to R⁶, one or two non-adjacent carbon atoms which are not bonded directly to the heteroatom may be replaced by atoms and/or atom groups selected from the group -O-, -S-, -S(O)-, -SO₂- or -P(O)R'-, where R' = non-, partially or perfluorinated C₁- to C₆-alkyl, C₃- to C₇-cycloalkyl, unsubstituted or substituted phenyl,
or of the formula (5)
[HetN]⁺ Cl⁻ (5),
where
HetN⁺ denotes a heterocyclic cation selected from the group 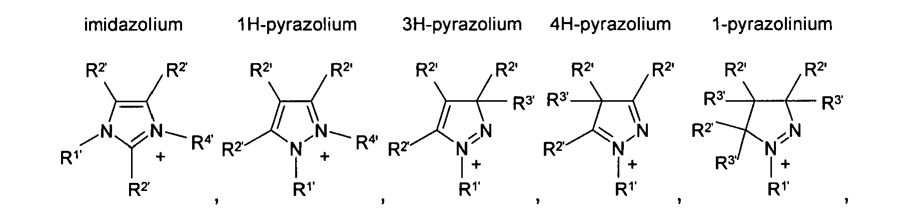 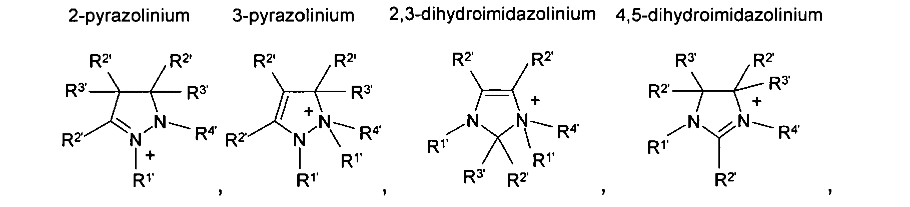 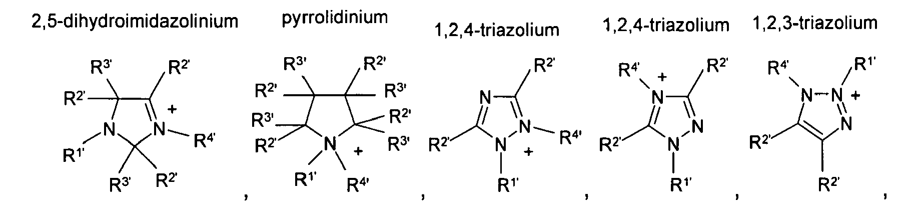 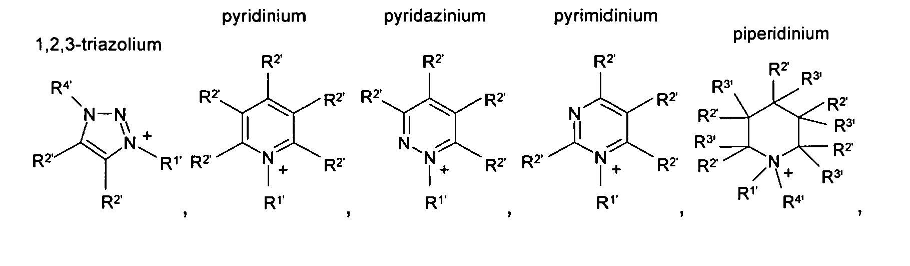 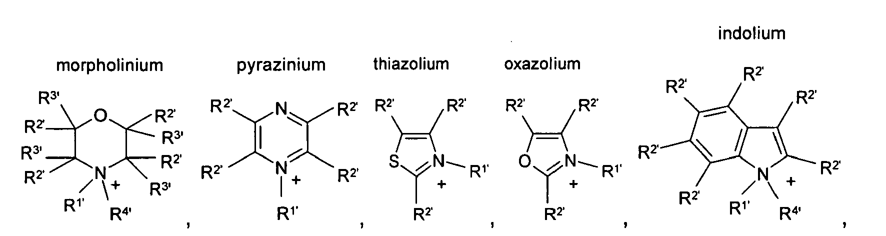 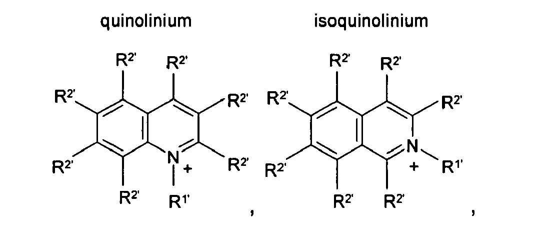 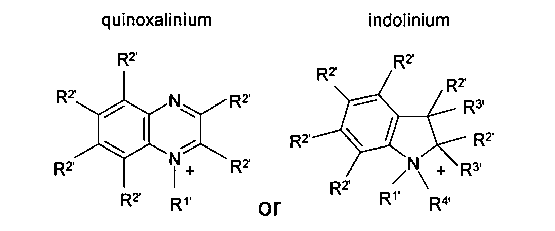 where the substituents
R^{1,} to R^{4,} each, independently of one another, denote hydrogen,
straight-chain or branched alkyl having 1-20 C atoms,
straight-chain or branched alkenyl having 2-20 C atoms and one or more double bonds,
straight-chain or branched alkynyl having 2-20 C atoms and one or more triple bonds,
saturated, partially or fully unsaturated cycloalkyl having 3-7 C atoms, which may be substituted by alkyl groups having 1-6 C atoms, saturated, partially or fully unsaturated heteroaryl, heteroaryl-C₁-C₆-alkyl or aryl-C₁-C₆-alkyl,
where the substituents R^{1'}, R^{2'}, R^{3'} and/or R^{4'} together may also form a ring system,
where one or more substituents R^{1,} to R^{4,} may be partially or fully substituted by halogens, in particular -F and/or -Cl, or partially by -NO₂, but where R^{1'} and R^{4'} cannot simultaneously be fully substituted by halogens,
and where, in the substituents R^{1'} to R^{4'}, one or two non-adjacent carbon atoms which are not bonded directly to the heteroatom may be replaced by atoms and/or atom groups selected from the group -O-, -S-, -S(O)-, -SO₂- or -P(O)R'-, where R' = non-, partially or perfluorinated C₁- to C₆-alkyl, C₃- to C₇-cycloalkyl, unsubstituted or substituted phenyl,
with an acid, where the reaction is carried out in water and the hydrochloric acid forming is removed by azeotropic distillation by coordination to 1,4-dioxane.

2. Process according to Claim 1, **characterised in that** an acid is selected from the group HBF₄, H₂SiF₆, H₂TiF₆, H₂ZrF₆, HSbF₆, HAsF₆, HPF₆, HN(CN)₂, HC(CN)₃, H₂SO₄, HNO₃, alkyl- or perfluoroalkylsulfonic acids, aromatic sulfonic acids, perfluoroalkylcarboxylic acids, alkyl- or perfluoro-alkylphosphinic acids, alkyl- or perfluoroalkylphosphonic acids, aromatic phosphinic or phosphonic acids or phosphoric acid.

3. Process according to Claim 1 or 2, **characterised in that** the azeotropic distillation is carried out batchwise, semicontinuously or continuously at atmospheric pressure or under reduced pressure.

## Revendications

1. Procédé de préparation de sels d'onium ayant une faible teneur en chlorure, par la réaction d'un chlorure d'onium de formule (1)
[NR₄]⁺ Cl⁻ (1),
ou de formule (2)
[PR₄]⁺ Cl⁻ (2),
dans lesquelles
R dans chaque cas, indépendamment les uns des autres, désigne H, où tous les substituants R ne peuvent pas simultanément être H, alkyle à chaîne linéaire ou ramifiée ayant 1-20 atomes de C,
alcényle à chaîne linéaire ou ramifiée ayant 2-20 atomes de C et
une ou plusieurs doubles liaisons,
alcynyle à chaîne linéaire ou ramifiée ayant 2-20 atomes de C et
une ou plusieurs triples liaisons,
cycloalkyle saturé ou partiellement ou totalement insaturé ayant 3-7 atomes de C, pouvant être substitué par des groupements alkyle ayant 1-6 atomes de C,
où un ou plusieurs R peuvent être partiellement ou totalement substitués par des halogènes, en particulier -F et/ou -Cl, ou partiellement par -NO₂, mais où tous les quatre ou trois R ne peuvent être totalement saturés par des halogènes,
et où un ou deux atomes de carbone non adjacents du substituant R qui ne sont pas en position α peuvent être remplacés par des atomes et/ou des groupes d'atomes sélectionnés parmi le groupe -O-, -S-, -S(O)-, -SO₂- ou -P(O)R'-, où R' = C₁- à C₆-alkyle non ou partiellement fluoré ou perfluoré, C₃- à C₇-cycloalkyle, phényle non substitué ou substitué,
ou de formule (3)
[(R¹R²N)-C(=SR⁷)(NR³R⁴)]⁺ Cl⁻ (3),
dans laquelle
chaque R¹ à R⁷, indépendamment les uns des autres, désigne hydrogène, où hydrogène est exclu pour R⁷,
alkyle à chaîne linéaire ou ramifiée ayant 1-20 atomes de C,
alcényle à chaîne linéaire ou ramifiée ayant 2-20 atomes de C et
une ou plusieurs doubles liaisons,
alcynyle à chaîne linéaire ou ramifiée ayant 2-20 atomes de C et
une ou plusieurs triples liaisons,
cycloalkyle saturé ou partiellement ou totalement insaturé ayant 3-7 atomes de C, pouvant être substitué par des groupements alkyle ayant 1-6 atomes de C,
où un ou plusieurs substituants R¹ à R⁷ peuvent être partiellement ou totalement substitués par des halogènes, en particulier -F et/ou -Cl, ou partiellement par -NO₂, mais où tous les substituants sur un atome de N ne peuvent être totalement substitués par des halogènes,
et où, dans les substituants R¹ à R⁶ un ou deux atomes de carbone non adjacents qui ne sont pas liés directement à l'hétéroatome peuvent être remplacés par des atomes et/ou des groupes d'atomes sélectionnés parmi le groupe -O-, -S-, -S(O)-, -SO₂- ou -P(O)R'-, où R' = C₁- à C₆-alkyle non ou partiellement fluoré ou perfluoré, C₃- à C₇-cycloalkyle, phényle non substitué ou substitué,
ou de formule (4)
[C(NR¹R²)(NR³R⁴)(NR⁵R⁶)]⁺ Cl⁻ (4),
dans laquelle
chaque R¹ à R⁶, indépendamment les uns des autres, désigne hydrogène,
alkyle à chaîne linéaire ou ramifiée ayant 1-20 atomes de C,
alcényle à chaîne linéaire ou ramifiée ayant 2-20 atomes de C et
une ou plusieurs doubles liaisons,
alcynyle à chaîne linéaire ou ramifiée ayant 2-20 atomes de C et
une ou plusieurs triples liaisons,
cycloalkyle saturé ou partiellement ou totalement insaturé ayant 3-7 atomes de C, pouvant être substitué par des groupements alkyle ayant 1-6 atomes de C,
où un ou plusieurs substituants R¹ à R⁶ peuvent être partiellement ou totalement substitués par des halogènes, en particulier -F et/ou -Cl, ou partiellement par -NO₂, mais où tous les substituants sur un atome de N ne peuvent être totalement substitués par des halogènes,
et où, dans les substituants R¹ à R⁶, un ou deux atomes de carbone non adjacents qui ne sont pas liés directement à l'hétéroatome peuvent être remplacés par des atomes et/ou des groupes d'atomes sélectionnés parmi le groupe -O-, -S-, -S(O)-, -SO₂- ou -P(O)R'-, où R' = C₁- à C₆-alkyle non ou partiellement fluoré ou perfluoré, C₃- à C₇-cycloalkyle, phényle non substitué ou substitué,
ou de formule (5)
[HétN]⁺ Cl⁻ (5),
dans laquelle
HétN⁺ désigne un cation hétérocyclique sélectionné parmi le groupe 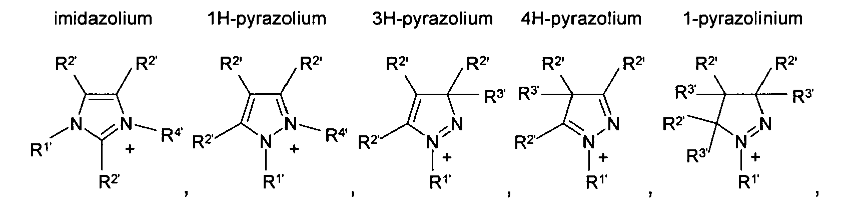 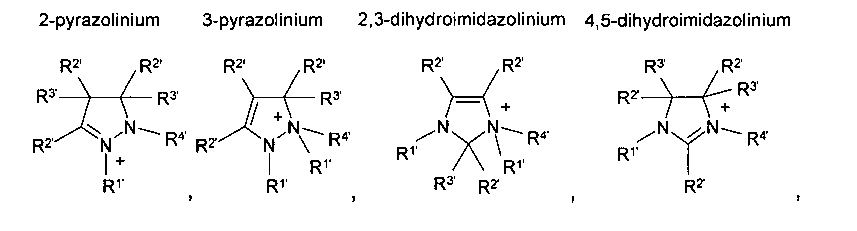 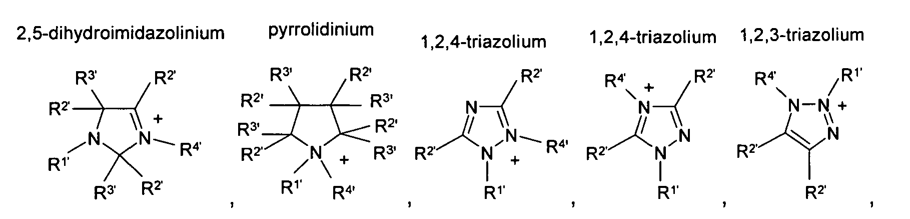 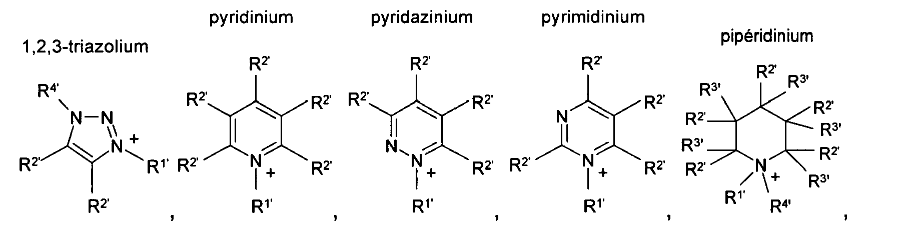 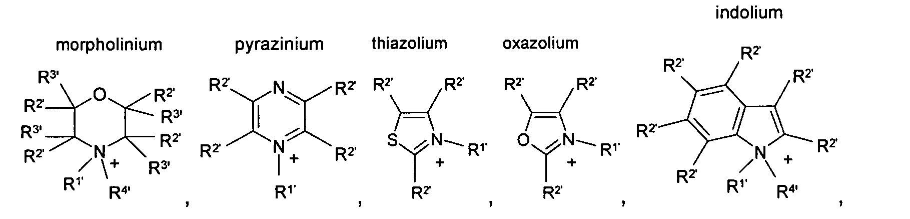 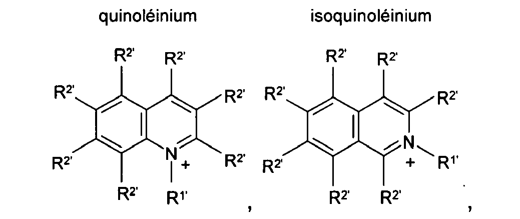 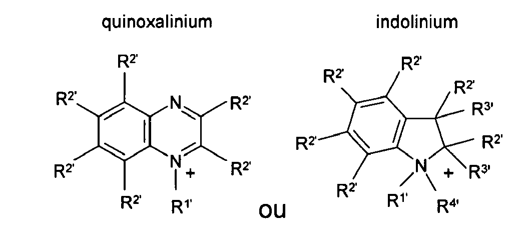 où les substituants
R^{1,} à R^{4,} chacun, indépendamment les uns des autres, désignent hydrogène,
alkyle à chaîne linéaire ou ramifiée ayant 1-20 atomes de C, alcényle à chaîne linéaire ou ramifiée ayant 2-20 atomes de C et une ou plusieurs doubles liaisons,
alcynyle à chaîne linéaire ou ramifiée ayant 2-20 atomes de C et une ou plusieurs triples liaisons,
cycloalkyle saturé ou partiellement ou totalement insaturé ayant 3-7 atomes de C, pouvant être substitué par des groupements alkyle ayant 1-6 atomes de C,
hétéroaryle saturé ou partiellement ou totalement insaturé, hétéroaryl-C₁-C₆-alkyle ou aryl-C₁-C₆-alkyle,
où les substituants R^{1'}, R^{2'}, R^{3'} et/ou R^{4'} ensemble peuvent également former un noyau,
où un ou plusieurs substituants R^{1,} à R^{4,} peuvent être partiellement ou totalement substitués par des halogènes, en particulier -F et/ou -Cl, ou partiellement par -NO₂, mais où R^{1'} et R^{4'} ne peuvent pas simultanément être totalement substitués par des halogènes,
et où, dans les substituants R^{1'} à R^{4'}, un ou deux atomes de carbone non adjacents qui ne sont pas liés directement à l'hétéroatome peuvent être remplacés par des atomes et/ou des groupes d'atomes sélectionnés parmi le groupe -O-, -S-, -S(O)-, -SO₂- ou -P(O)R'-, où R' = C₁- à C₆-alkyle non ou partiellement fluoré ou perfluoré, C₃- à C₇-cycloalkyle, phényle non substitué ou substitué,
avec un acide, où la réaction est réalisée dans l'eau et l'acide chlorhydrique formé est éliminé par distillation azéotropique par coordination au 1,4-dioxane.

2. Procédé selon la revendication 1, **caractérisé en ce qu'**un acide est sélectionné parmi le groupe HBF₄, H₂SiF₆, H₂TiF₆, H₂ZrF₆, HSbF₆, HAsF₆, HPF₆, HN(CN)₂, HC(CN)₃, H₂SO₄, HNO₃, les acides alkyl- ou perfluoroalkylsulfoniques, les acides sulfoniques aromatiques, les acides perfluoroalkylcarboxyliques, les acides alkyl- ou perfluoroalkylphosphiniques, les acides alkyl- ou perfluoroalkylphosphoniques, les acides phosphiniques ou phosphoniques aromatiques ou l'acide phosphorique.

3. Procédé selon la revendication 1 ou 2, **caractérisé en ce que** la distillation azéotropique est réalisée en mode discontinu, semi-continu ou continu à pression atmosphérique ou sous pression réduite.
